# EUROPEAN PATENT APPLICATION

(11) **EP 4 458 855 A1**
(43) Date of publication of application: **06.11.2024**
(21) Application number: 22915220.2
(22) Date of filing: 30.12.2022
(51) Int. Cl.: C07K 16/28, C12N 15/13, C12N 15/85, A61K 39/395, A61K 51/10, A61K 35/00

(54) **DEVELOPMENT OF NOVEL PDL1 SINGLE-DOMAIN ANTIBODY**

(30) Priority: 31.12.2021 CN 202111672054
(71) Applicant: Persongen Biotherapeutics (Suzhou) Co., Ltd., Suzhou, Jiangsu 215000 (CN)
(72) Inventor: YANG, Lin, Suzhou, Jiangsu 215000 (CN); YOU, Fengtao, Suzhou, Jiangsu 215000 (CN)
(74) Representative: dompatent von Kreisler Selting Werner - Partnerschaft von Patent- und Rechtsanwälten mbB
(86) International application number: PCT/CN2022/144146
(87) International publication number: WO 2023/125973

(57) **Abstract**

Disclosed are a nanobody for PDL1 and a use thereof. Provided are a nanobody for PDL1 and a sequence thereof. Also provided are a polynucleotide encoding the nanobody, a corresponding expression vector, a host cell capable of expressing the nanobody, and a method of producing the nanobody. The nanobody can specifically bind to human PDL1 and has a high affinity as well as an excellent PD-1/PDL1 blocking activity. The nanobody can block only PD-1 and PDL1 pathways without affecting PD-1 and PD-L2 pathways, thereby avoiding side effects such as interstitial pneumonia as well as increasing safety. The nanobody can also more comprehensively activate the immune system to kill tumors, and the curative effect can be achieved for patients for whom PD-1 is ineffective or who are resistant to PD-1. The PDL1 nanobody can not only inhibit the PD-1-PDL1 pathway but also inhibit the co-inhibition function of B7.1 and PDL1, so that the T cell function and cell factor generation can be comprehensively activated, thereby achieving an improved immune effect.

## Description

### TECHNICAL FIELD

The present invention relates to the field of biomedical or biopharmaceutical technology. Specifically, the present invention relates to a single-domain antibody specifically targeting PDL1 and a use thereof.

### BACKGROUND

PD-1/PDL1 immunotherapy is one of the most successful tumor immunotherapies, which is setting off a revolution in tumor treatment and transforming the landscape of cancer treatment. Immune checkpoint inhibitors are designed to make full use of the human body's own immune system to resist and fight against cancer, and kill cancer cells by blocking the PD-1/PDL1 signaling pathway. They have the potential to treat various types of tumors, substantially improve the survival of patients with advanced tumors, and become a "specific" drug for tumor patients.

Currently, there have been several PD-1 antibodies and PD-1 inhibitors commercially available both domestically and internationally. Similar to PD-1 antibodies, PDL1 antibodies can block the immune suppression mediated by the binding of PDL1 to PD-1 on the surface of T cells and reactivate T cells to recognize and kill tumor cells, thereby inhibiting tumor growth. Unlike PD-1 monoclonal antibodies, PDL1 monoclonal antibodies bind to PDL1 expressed on tumor cells or tumor-infiltrating immune cells, while PD-1 monoclonal antibodies bind to PD-1.

However, the current limitations of PD-1 antibodies are: (1) In addition to blocking the PD-1 and PDL1 pathways, PD-1 antibodies may also affect the PD-1 and PD-L2 pathways, potentially leading to side effects such as interstitial pneumonia; (2) Some patients have ineffective treatment with PD-1 monoclonal antibody or develop resistance to PD-1 monoclonal antibody, for whom alternative drugs are needed; (3) The efficacy and safety of PD-1 antibodies still need improvement.

Therefore, there is an urgent need in this field to develop a novel drug for inhibiting the PD-1/PDL1 signaling pathway that can achieve better therapeutic effects, have a wider range of applications, and be safer.

### SUMMARY OF THE INVENTION

The purpose of the present invention is to provide a PDL1-targeting nanobody and a use thereof.

In the first aspect of the present invention, it provides a PDL1-targeting nanobody.

In another preferred embodiment, the PDL1-targeting nanobody is capable of specifically binding to PDL1.

In another preferred embodiment, the complementarity determining regions (CDRs) of the PDL1-targeting nanobody is one or more selected from the group consisting of:
(1) CDR1 as shown in SEQ ID NO: 7, CDR2 as shown in SEQ ID NO: 8, and CDR3 as shown in SEQ ID NO: 9;
(2) CDR1 as shown in SEQ ID NO: 10, CDR2 as shown in SEQ ID NO: 11, and CDR3 as shown in SEQ ID NO: 12;
(3) CDR1 as shown in SEQ ID NO: 10, CDR2 as shown in SEQ ID NO: 13, and CDR3 as shown in SEQ ID NO: 14;
(4) CDR1 as shown in SEQ ID NO: 10, CDR2 as shown in SEQ ID NO: 13, and CDR3 as shown in SEQ ID NO: 15;
(5) CDR1 as shown in SEQ ID NO: 7, CDR2 as shown in SEQ ID NO: 16, and CDR3 as shown in SEQ ID NO: 17;
(6) CDR1 as shown in SEQ ID NO: 18, CDR2 as shown in SEQ ID NO: 19, and CDR3 as shown in SEQ ID NO: 20.

In another preferred embodiment, any one of the above amino acid sequences further comprises a derivative sequence that is optionally added, deleted, modified and/or substituted with at least one (such as 1-3, preferably 1-2, more preferably 1) amino acid and is capable of retaining the ability to specifically bind to PDL1.

In another preferred embodiment, the derivative sequence that is added, deleted, modified and/or substituted with at least one amino acid and is capable of retaining the ability to specifically bind to PDL1 is an amino acid sequence with a homology or sequence identity of at least 85%, at least 90%, at least 95%, at least 96%, at least 97%, at least 98%, or at least 99%.

In another preferred embodiment, the CDR1, CDR2 and CDR3 are separated by framework regions FR1, FR2, FR3 and FR4 of a VHH chain.

In another preferred embodiment, the PDL1-targeting nanobody further comprises framework regions (FRs).

In another preferred embodiment, the framework regions (FRs) are derived from amino acid sequences as shown in SEQ ID NOs: 1-6.

In another preferred embodiment, the framework regions (FRs) is one or more selected from the group consisting of:
(1) FR1 as shown in SEQ ID NO: 21, FR2 as shown in SEQ ID NO: 22, FR3 as shown in SEQ ID NO: 23, and FR4 as shown in SEQ ID NO: 24;
(2) FR1 as shown in SEQ ID NO: 25, FR2 as shown in SEQ ID NO: 26, FR3 as shown in SEQ ID NO: 27, and FR4 as shown in SEQ ID NO: 24;
(3) FR1 as shown in SEQ ID NO: 28, FR2 as shown in SEQ ID NO: 29, FR3 as shown in SEQ ID NO: 30, and FR4 as shown in SEQ ID NO: 24;
(4) FR1 as shown in SEQ ID NO: 31, FR2 as shown in SEQ ID NO: 29, FR3 as shown in SEQ ID NO: 32, and FR4 as shown in SEQ ID NO: 24;
(5) FR1 as shown in SEQ ID NO: 33, FR2 as shown in SEQ ID NO: 22, FR3 as shown in SEQ ID NO: 34, and FR4 as shown in SEQ ID NO: 24;
(6) FR1 as shown in SEQ ID NO: 35, FR2 as shown in SEQ ID NO: 36, FR3 as shown in SEQ ID NO: 37, and FR4 as shown in SEQ ID NO: 24.

In another preferred embodiment, the amino acid sequence of the VHH chain of the PDL1-targeting nanobody is selected from the group consisting of: SEQ ID NO: 1, SEQ ID NO: 2, SEQ ID NO: 3, SEQ ID NO: 4, SEQ ID NO: 5, SEQ ID NO: 6, and a combination thereof.

In another preferred embodiment, the PDL1-targeting nanobody includes a humanized antibody, a camel-derived antibody, or a chimeric antibody.

In another preferred embodiment, the PDL1-targeting nanobody is a llama-derived antibody.

In the second aspect of the present invention, it provides a PDL1-targeting antibody, which comprises one or more VHH chain(s) of the PDL1-targeting nanobody of the first aspect of the present invention.

In another preferred embodiment, the amino acid sequence of the VHH chain of the PDL1-targeting nanobody is selected from the group consisting of: SEQ ID NO: 1, SEQ ID NO: 2, SEQ ID NO: 3, SEQ ID NO: 4, SEQ ID NO: 5, SEQ ID NO: 6, and a combination thereof.

In another preferred embodiment, the PDL1-targeting antibody may be a monomer, a bivalent antibody, and/or a multivalent antibody.

In another preferred embodiment, the PDL1-targeting antibody is a bivalent antibody.

In the third aspect of the present invention, it provides a chimeric antigen receptor (CAR), which comprises an extracellular domain, wherein the extracellular domain comprises the PDL1-targeting nanobody of the first aspect of the present invention, or the PDL1-targeting antibody of the second aspect of the present invention.

In another preferred embodiment, the extracellular domain further comprises a signal peptide.

In another preferred embodiment, the extracellular domain further comprises other exogenous proteins.

In another preferred embodiment, the antibody contained in the extracellular domain has an amino acid sequence as shown in SEQ ID NOs: 1-6.

In another preferred embodiment, the antibody contained in the extracellular domain has an amino acid sequence with a homology of ≥85% (preferably ≥90%, more preferably ≥95%) to SEQ ID NOs: 7-17, or has an amino acid sequence that differs from SEQ ID NOs: 1-6 by 1, 2, or 3 amino acids.

In another preferred embodiment, the CAR has a structure as shown in formula Ia:

L1-Nb-H-TM-C-CD3ζ (Ia)

wherein,
L is absent or a signal peptide sequence;
Nb is a specific binding domain;
H is absent or a hinge region;
TM is a transmembrane domain;
C is a costimulatory signaling domain;
CD3ζ is a cytoplasmic signaling sequence derived from CD3ζ (including wild-type, or a mutant/modified variant thereof);
"-" represents a linker peptide or peptide bond.

In another preferred embodiment, L is a signal peptide of a protein selected from the group consisting of: CD8, GM-CSF, CD4, CD28, CD137, and a mutant/modified variant thereof, and a combination thereof.

In another preferred embodiment, Nb targets PDL1.

In another preferred embodiment, Nb is a PDL1 nanobody.

In another preferred embodiment, H is a hinge region of a protein selected from the group consisting of: CD8, CD28, CD137, IgG, and a combination thereof.

In another preferred embodiment, H is a human IgG1 Fc hinge region.

In another preferred embodiment, TM is a transmembrane region of a protein selected from the group consisting of: CD28, CD3 epsilon, CD45, CD4, CD5, CD8, CD9, CD16, CD22, CD33, CD37, CD64, CD80, CD86, CD134, CD137, CD154, CD278, CD152, CD279, CD233, and a mutant/modified variant thereof, and a combination thereof.

In another preferred embodiment, C is a costimulatory domain of a protein selected from the group consisting of: OX40, CD2, CD7, CD27, CD28, CD30, CD40, CD70, CD134, 4-1BB (CD137), PD-1, Dap10, LIGHT, NKG2C, B7-H3, ICAM-1, LFA-1 (CD11a/CD18), ICOS (CD278), NKG2D, GITR, OX40L, 2B4, TLR, and a mutant/modified variant thereof, and a combination thereof.

In the fourth aspect of the present invention, it provides a recombinant protein, which comprises:
(i) the PDL1-targeting nanobody of the first aspect of the present invention, or the PDL1-targeting antibody of the second aspect of the present invention, or the chimeric antigen receptor of the third aspect of the present invention; and
(ii) optionally a tag sequence for assisting expression and/or purification.

In another preferred example, the tag sequence includes an Fc tag, an HA tag, a GGGS sequence, a FLAG tag, a Myc tag, a 6His tag, and a combination thereof.

In another preferred embodiment, the recombinant protein specifically binds to PDL1.

In another preferred embodiment, the recombinant protein (or polypeptide) includes a fusion protein.

In another preferred embodiment, the recombinant protein is a monomer, a dimer, or a multimer.

In another preferred embodiment, the recombinant protein specifically binds to PDL1.

In another preferred embodiment, the tag sequence is an Fc tag.

In the fifth aspect of the present invention, it provides a polynucleotide encoding a protein selected from the group consisting of: the PDL1-targeting nanobody of the first aspect of the present invention, or the PDL1-targeting antibody of the second aspect of the present invention, or the chimeric antigen receptor of the third aspect of the present invention, or the recombinant protein of the fourth aspect of the present invention.

In another preferred embodiment, the polynucleotide encodes a protein selected from the group consisting of: the PDL1-targeting nanobody of the first aspect of the present invention, or the PDL1-targeting antibody of the second aspect of the present invention.

In another preferred embodiment, the present invention relates to a nucleic acid molecule encoding the PDL1-targeting nanobody of the present invention. The nucleic acid molecule of the present invention may be RNA, DNA or cDNA.

In the sixth aspect of the present invention, it provides an expression vector comprising the polynucleotide of the fifth aspect of the present invention.

In another preferred embodiment, the expression vector is selected from the group consisting of: DNA, RNA, a viral vector, a plasmid, a transposon, other gene transfer system, and a combination thereof. Preferably, the expression vector comprises a viral vector, such as lentivirus, adenovirus, AAV virus, retrovirus, and a combination thereof.

In another preferred embodiment, the expression vector is selected from the group consisting of: a pTomo lentivirus vector, plenti, pLVTH, pLJM1, pHCMV, pLBS.CAG, pHR, pLV, etc.

In another preferred embodiment, the expression vector is a pcDNA3.4-hIgG1-Fc2 vector.

In another preferred embodiment, the expression vector further comprises an element selected from the group consisting of: a promoter, a transcription enhancing element WPRE, a long terminal repeat (LTR) sequence, etc.

In the seventh aspect of the present invention, it provides a host cell comprising the expression vector of the sixth aspect of the present invention, or having the polynucleotide of the fifth aspect of the present invention integrated into its genome.

In another preferred embodiment, the host cell comprises a prokaryotic cell or a eukaryotic cell.

In another preferred embodiment, the host cell is selected from the group consisting of: *Escherichia coli*, a yeast cell, and a mammalian cell.

In another preferred embodiment, the host cell is a 293F cell.

In the eighth aspect of the present invention, it provides an engineered immune cell comprising the expression vector of the sixth aspect of the present invention, or having the polynucleotide of the fifth aspect of the present invention integrated into its genome.

In another preferred embodiment, the engineered immune cell is selected from the group consisting of:
(i) a chimeric antigen receptor αβT cell (a CAR-T cell);
(ii) a chimeric antigen receptor γδT cell (a CAR-T cell);
(iii) a chimeric antigen receptor NKT cell (a CAR-NKT cell);
(iv) a chimeric antigen receptor NK cell (a CAR-NK cell).

In another preferred embodiment, the engineered immune cell comprises an autologous or allogeneic αβT cell, γδT cell, NKT cell, NK cell, or a combination thereof.

In another preferred embodiment, the engineered immune cell is a CAR-T cell.

In the ninth aspect of the present invention, it provides a method for producing a PDL1-targeting nanobody, which comprises the steps of:
(a) culturing the host cell of the seventh aspect of the present invention under conditions suitable for producing a nanobody, thereby obtaining a culture containing the PDL1-targeting nanobody;
(b) isolating and/or recovering the PDL1-targeting nanobody from the culture; and
(c) optionally, purifying and/or modifying the PDL1-targeting nanobody obtained in step (b).

In the tenth aspect of the present invention, it provides a method for producing the engineered immune cell of the eighth aspect of the present invention, which comprises the step of: transducing the polynucleotide of the fifth aspect of the present invention or the expression vector of the sixth aspect of the present invention into an immune cell, thereby obtaining the engineered immune cell.

In another preferred embodiment, the method further comprises the step of performing functional and efficacy testing on the obtained engineered immune cell.

In the eleventh aspect of the present invention, it provides an immunoconjugate, which comprises:
(a) the PDL1-targeting nanobody of the first aspect of the present invention, or the PDL1-targeting antibody of the second aspect of the present invention, or the recombinant protein of the fourth aspect of the present invention; and
(b) a coupling moiety selected from the group consisting of: a detectable label, a drug, a cytokine, a radionuclide, an enzyme, a gold nanoparticle/nanorod, a nanomagnetic particle, a viral coat protein or VLP, and a combination thereof.

In another preferred embodiment, the (a) moiety is the PDL1-targeting nanobody of the first aspect of the present invention, or the PDL1-targeting antibody of the second aspect of the present invention.

In another preferred embodiment, the (a) moiety is coupled with the coupling moiety through a chemical bond or a linker.

In another preferred embodiment, the radionuclide comprises:
(i) a diagnostic isotope, which is selected from the group consisting of: Tc-99m, Ga-68, F-18, 1-123, 1-125, I-131, In-111, Ga-67, Cu-64, Zr-89, C-11, Lu-177, Re-188, and a combination thereof; and/or
(ii) a therapeutic isotope, which is selected from the group consisting of: Lu-177, Y-90, Ac-225, As-211, Bi-212, Bi-213, Cs-137, Cr-51, Co-60, Dy-165, Er-169, Fm-255, Au-198, Ho-166, I-125, 1-131, Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133 Yb-169, Yb-177, and a combination thereof.

In another preferred embodiment, the coupling moiety is a drug or toxin.

In another preferred embodiment, the drug is a targeted therapeutic drug for a PDL1-high expressing disease.

In another preferred embodiment, the PDL1-high expressing disease is selected from the group consisting of: gastric cancer, lung cancer, liver cancer, osteosarcoma, breast cancer, pancreatic cancer, lymphoma, etc.

In another preferred embodiment, the drug is a cytotoxic drug.

In another preferred embodiment, the cytotoxic drug is selected from the group consisting of: an antitubulin drug, a DNA minor groove binding agent, a DNA replication inhibitor, an alkylating agent, an antibiotic, a folate antagonist, an antimetabolite drug, a chemotherapeutic sensitizer, a topoisomerase inhibitor, a vinca alkaloid, and a combination thereof.

Examples of particularly useful cytotoxic drug classes include, for example, DNA minor groove binding agents, DNA alkylating agents, and tubulin inhibitors. Typical cytotoxic drugs include, for example, Auristatins, Camptothecins, Duocarmycins, Etoposides, Maytansines, and Maytansinoids (such as DM1 and DM4), Taxanes, Benzodiazepines, or Benzodiazepine containing drugs (such as Pyrrolo[1,4] benzodiazepines (PBDs), Indolinobenzodiazepines, and Oxazolidinobenzodiazepines), Vinca alkaloids, and combinations thereof.

In another preferred embodiment, the toxin is selected from the group consisting of: auristatins (such as auristatin E, auristatin F, MMAE, and MMAF), aureomycin, maytansinoids, ricin, ricin A-chain, combretastatin, duocarmycin, dolastatin, doxorubicin, daunorubicin, paclitaxel, cisplatin, cc1065, ethidium bromide, mitomycin, etoposide, tenoposide, vincristine, vinblastine, colchicine, dihydroxy anthracine dione, actinomycin, diphtheria toxin, pseudomonas exotoxin (PE) A, PE40, abrin, abrin A chain, modeccin A chain, α-sarcina, gelonin, mitogellin, restrictocin, phenomycin, enomycin, curicin, crotin, calicheamicin, saponaria officinalis inhibitor, glucocorticoid, and a combination thereof.

In another preferred embodiment, the coupling moiety is a detectable label.

In another preferred embodiment, the coupling moiety is selected from the group consisting of: a fluorescent or luminescent label, a radioactive label, MRI (magnetic resonance imaging) or CT (electronic computer X-ray tomography technique) contrast agent, or an enzyme capable of producing a detectable product, a radionuclide, a biotoxin, a cytokine (such as IL-2, etc.), an antibody, an Fc fragment of an antibody, an scFv fragment of an antibody, a gold nanoparticle/nanorod, a viral particle, a liposome, a nanomagnetic particle, a prodrug activating enzyme (such as DT-diaphorase (DTD) or biphenyl hydrolase-like protein (BPHL)), or a nanoparticle in any form.

In another preferred embodiment, the immunoconjugate comprises: a multivalent (such as bivalent) VHH chain of the PDL1-targeting nanobody of the first aspect of the present invention.

In another preferred embodiment, the multivalent refers that the amino acid sequence of the immunoconjugate comprises multiple identical or different repeated VHH chains of the PDL1-targeting nanobody of the first aspect of the present invention.

In the twelfth aspect of the present invention, it provides a use of an active ingredient selected from the group consisting of: the PDL1-targeting nanobody of the first aspect of the present invention, or the PDL1-targeting antibody of the second aspect of the present invention, or the chimeric antigen receptor of the third aspect of the present invention, or the recombinant protein of the fourth aspect of the present invention, or the engineered immune cell of the eighth aspect of the present invention, or the immunoconjugate of the eleventh aspect of the present invention, and a combination thereof, wherein the active ingredient is used for preparing:
(1) a drug for preventing and/or treating a PDL1-high expressing disease; and/or
(2) a reagent for detecting a PDL1-high expressing disease.

In another preferred embodiment, the reagent is a diagnostic reagent, and preferably the diagnostic reagent is a detection slide or a detection plate.

In another preferred embodiment, the diagnostic reagent is used for: detecting PDL1 protein or a fragment thereof in a sample.

In another preferred embodiment, the PDL1-high expressing disease is selected from the group consisting of: gastric cancer, lung cancer, liver cancer, osteosarcoma, breast cancer, pancreatic cancer, lymphoma, etc.

In the thirteenth aspect of the present invention, it provides a method for *in vitro* detection of PDL1 protein or a fragment thereof in a sample, wherein the method comprises the steps of:
(1) contacting the sample *in vitro* with the PDL1-targeting nanobody of the first aspect of the present invention, or the PDL1-targeting antibody of the second aspect of the present invention, or the chimeric antigen receptor of the third aspect of the present invention, or the recombinant protein of the fourth aspect of the present invention, or the engineered immune cell of the eighth aspect of the present invention, or the immunoconjugate of the eleventh aspect of the present invention, or a combination thereof;
(2) detecting whether an antigen-antibody complex is formed, wherein the formation of the complex indicates the presence of PDL1 protein or a fragment thereof in the sample.

In another preferred embodiment, the detection includes a diagnostic detection and a non-diagnostic detection.

In the fourteenth aspect of the present invention, it provides a pharmaceutical composition, which comprises:
(a) the PDL1-targeting nanobody of the first aspect of the present invention, or the PDL1-targeting antibody of the second aspect of the present invention, or the chimeric antigen receptor of the third aspect of the present invention, or the recombinant protein of the fourth aspect of the present invention, or the engineered immune cell of the eighth aspect of the present invention, or the immunoconjugate of the eleventh aspect of the present invention, or a combination thereof, as an active ingredient; and
(ii) a pharmaceutically acceptable carrier, diluent, or excipient.

In another preferred embodiment, the dosage form of the pharmaceutical composition is selected from the group consisting of: an injectionand a lyophilization agent.

In another preferred embodiment, the pharmaceutical composition comprises 0.01-99.99% of the PDL1-targeting nanobody of the first aspect of the present invention, or the PDL1-targeting antibody of the second aspect of the present invention, or the chimeric antigen receptor of the third aspect of the present invention, or the recombinant protein of the fourth aspect of the present invention, or the engineered immune cell of the eighth aspect of the present invention, or the immunoconjugate of the eleventh aspect of the present invention, or a combination thereof, and 0.01-99.99% of the pharmaceutically acceptable carrier, wherein the percentage is a mass percentage of the pharmaceutical composition.

In another preferred embodiment, the concentration of the engineered immune cell in the active ingredient is 1×10³-1×10⁸ cells/mL, preferably 1×10⁴-1×10⁷ cells/mL.

In the fifteenth aspect of the present invention, it provides a kit, wherein the kit comprises:
(1) a first container, wherein the first container comprises the PDL1-targeting nanobody of the first aspect of the present invention, or the PDL1-targeting antibody of the second aspect of the present invention, or the chimeric antigen receptor of the third aspect of the present invention, or the recombinant protein of the fourth aspect of the present invention, or the engineered immune cell of the eighth aspect of the present invention, or the immunoconjugate of the eleventh aspect of the present invention, or a combination thereof; and/or
(2) a second container, wherein the second container comprises a secondary antibody against the content of the first container;
   or,
   the kit comprises a detection plate comprising: a substrate (support plate) and a test strip, wherein the test strip comprises the PDL1-targeting nanobody of the first aspect of the present invention, or the PDL1-targeting antibody of the second aspect of the present invention, or the recombinant protein of the third aspect of the present invention, or the immunoconjugate of the eighth aspect of the present invention, or a combination thereof.

In another preferred embodiment, the kit further comprises instructions, and according to the instructions, the kit is used for non-invasive detection of PDL1 expression in a subject to be tested.

In another preferred embodiment, the kit is used for the detection of a PDL1-high expressing disease.

In another preferred embodiment, the PDL1-high expressing disease is selected from the group consisting of: gastric cancer, lung cancer, liver cancer, osteosarcoma, breast cancer, pancreatic cancer, lymphoma, etc.

In the sixteenth aspect of the present invention, it provides a method of preventing and/or treating a PDL1-high expressing disease, which comprises a step of: administering the PDL1-targeting nanobody of the first aspect of the present invention, or the PDL1-targeting antibody of the second aspect of the present invention, or the chimeric antigen receptor of the third aspect of the present invention, or the recombinant protein of the fourth aspect of the present invention, or the engineered immune cell of the eighth aspect of the present invention, or the immunoconjugate of the eleventh aspect of the present invention, or the pharmaceutical composition of the fourteenth aspect of the present invention, or a combination thereof, to a subject in need thereof.

In another preferred embodiment, the subject comprises a mammal, such as a human.

In another preferred embodiment, the PDL1-high expressing disease is selected from the group consisting of: gastric cancer, lung cancer, liver cancer, osteosarcoma, breast cancer, pancreatic cancer, lymphoma, etc.

In another preferred embodiment, a CAR immune cell contained in the engineered immune cell or the pharmaceutical composition is derived from a cell of the subject (an autologous cell).

In another preferred embodiment, a CAR immune cell contained in the engineered immune cell or the pharmaceutical composition is derived from a cell of a healthy individual (an allogeneic cell).

In another preferred embodiment, the method may be used in combination with other treatment methods.

In another preferred embodiment, the other treatment methods comprise chemotherapy, radiotherapy, targeted therapy, etc.

In the seventeenth aspect of the present invention, it provides a method for diagnosis of a PDL 1-high expressing disease, which comprises the steps of:
(i) collecting a sample from a subject to be diagnosed, contacting the sample with the PDL1-targeting nanobody of the first aspect of the present invention, or the PDL1-targeting antibody of the second aspect of the present invention, or the chimeric antigen receptor of the third aspect of the present invention, or the recombinant protein of the fourth aspect of the present invention, or the engineered immune cell of the eighth aspect of the present invention, or the immunoconjugate of the eleventh aspect of the present invention, or a combination thereof; and
(ii) detecting whether an antigen-antibody complex is formed, wherein the formation of the complex indicates that the subject is a confirmed patient with the PDL1-high expression disease.

In another preferred embodiment, the sample is a blood sample or a throat swab sample, or a sample from other tissues or organs.

In another preferred embodiment, the PDL1-high expression disease is selected from the group consisting of: gastric cancer, lung cancer, liver cancer, osteosarcoma, breast cancer, pancreatic cancer, lymphoma, etc.

In the eighteenth aspect of the present invention, it provides a method for preparing a recombinant polypeptide which is the PDL1-targeting nanobody of the first aspect of the present invention, or the PDL1-targeting antibody of the second aspect of the present invention, or the chimeric antigen receptor of the third aspect of the present invention, or the recombinant protein of the fourth aspect of the present invention, wherein the method comprises the steps of:
(a) culturing the host cell of the fifth aspect of the present invention under conditions suitable for expression; and
(b) isolating the recombinant polypeptide from the culture.

### DESCRIPTION OF DRAWINGS

Figure 1 shows the results of SDS-PAGE identification of PDL1-Fc protein.
Figure 2 shows the ELISA detection results of immune serum.
Figure 3 shows the agarose gel electrophoresis results for detecting PCR products, wherein Figure 3A shows that two PCR bands, of approximately 1000 bp and 750 bp, were obtained from the first round of PCR, and the 750 bp fragments were recovered from the gel as templates for the second round of PCR; Figure 3B shows that the second round of PCR obtained bands of about 450 bp, which were identified as VHH fragments by agarose gel electrophoresis.
Figure 4 shows the alignment results of the phage display library sequences.
Figure 5 to Figure 9 show the phage ELISA results of solid-phase panning products from the phage display library, wherein red indicates clones that bind to both PDL1 antigen and Fc; blue indicates clones that bind only to the PDL1 target antigen but not to Fc; and green indicates clones that bind only to the PDL1 target antigen, but not to Fc, and have strong binding to the PDL1 target antigen.
Figure 10 and Figure 11 show the flow cytometry detection results of products obtained by transient transfection using the overlap products.
Figure 12 to Figure 14 show the flow cytometry detection of the binding of purified antibodies to CHO-K1/PDL1, a recombinant cell line overexpressing PDL1.
Figure 15 shows the detection results of PD-1-PDL1 blocking activity of monovalent antibodies.
Figure 16 shows the detection results of PD-1-PDL1 blocking activity of bivalent antibodies.

### DETAILED DESCRIPTION

After extensive and intensive researches and massive screening, the inventors have developed multiple PDL1-targeting nanobodies and the bivalent antibodies constructed from them for the first time. Specifically, the present invention utilized human-derived PDL1 protein to immunize llamas and obtained a high-quality immune nanobody gene library. Subsequently, PDL1 protein molecules were coupled onto an enzyme-linked immunosorbent assay plate, and by using the principle of antigen-antibody specific binding, the antigens in this form were used to screen for an immune nanobody gene library (llama heavy chain antibody phage display gene library) via phage display technology, thereby obtaining PDL1-specific nanobody genes. In addition, relevant experimental results show that the PDL1-targeting nanobodies obtained by the present invention, as well as the bivalent antibodies constructed from them, can effectively bind to the PDL1 protein and have blocking function for blocking the interaction with their ligands PD-1. On this basis, the present invention has been completed. The PDL1-targeting nanobodies developed by the present invention can be used as a new therapeutic method for targeted therapy of gastric cancer, lung cancer, liver cancer, osteosarcoma, breast cancer, pancreatic cancer, lymphoma, etc.

### Terms

As used herein, the terms "antibody of the invention", "antibody of the present invention", "PDL1-targeting nanobody of the present invention", "PDL1-targeting nanobody of the invention", "PDL1-targeting nanobody", and "nanobody against PDL1" have the same meaning and can be used interchangeably to refer to an antibody that specifically recognizes and binds to PDL1 proteins (including human PDL1 proteins).

The antibody numbers and corresponding sequence numbers of the nanobodies of the present invention are shown in Table 1.

**Table 1**

| Antibody number | CDR1 | CDR2 | CDR3 | FR1 | FR2 | FR3 | FR4 | Amino acid sequence number of VHH chain |
|---|---|---|---|---|---|---|---|---|
| 17-A06 | 7 | 8 | 9 | 21 | 22 | 23 | 24 | 1 |
| 17-C12 | 10 | 11 | 12 | 25 | 26 | 27 | 24 | 2 |
| 17-A01 | 10 | 13 | 14 | 28 | 29 | 30 | 24 | 3 |
| 17-G06 | 10 | 13 | 15 | 31 | 29 | 32 | 24 | 4 |
| 23-G3 | 7 | 16 | 17 | 33 | 22 | 34 | 24 | 5 |
| 29-H9 | 18 | 19 | 20 | 35 | 36 | 37 | 24 | 6 |

| | | | | | | | | |
|---|---|---|---|---|---|---|---|---|
| Note: The values in the table represent sequence numbers, i.e., "1" represents "SEQ ID NO: 1". The sequence numbers of CDR1, CDR2, CDR3, FR1, FR2, FR3, and FR4 displayed in the table are the amino acid sequence numbers thereof. | | | | | | | | |

As used herein, the term "antibody" or "immunoglobulin" as used herein refers to a heterotetrameric glycoprotein having the same structural feature of about 150,000 daltons, consisting of two identical light chains (L) and two identical heavy chains (H). Each light chain is linked to a heavy chain by a covalent disulfide bond, and the numbers of disulfide bonds between the heavy chains of different immunoglobulin isoforms are different. Each heavy and light chain also has regularly spaced intrachain disulfide bonds. One end of each heavy chain has a variable region (VH) followed by a plurality of constant regions. There is a variable region (VL) at one end of each chain and a constant region at the other end; the constant region of the light chain corresponds to the first constant region of the heavy chain; and the variable region of the light chain corresponds to the variable region of the heavy chain. An interface is formed between the variable regions of the light and heavy chains by particular amino acid residues.

As used herein, the terms "single-domain antibody", "VHH", "nanobody", "single domain antibody (sdAb, or nanobody)" have the same meaning and can be used interchangeably to refer to a single-domain antibody (VHH) consisting of only one heavy chain variable region, which is constructed by cloning the heavy chain variable region of an antibody. It is the smallest antigen-binding fragment with complete function. Usually, the antibody with natural deletion of light chain and heavy chain constant region 1 (CH1) is obtained first, and then the heavy chain variable region of the antibody is cloned to construct a single-domain antibody (VHH) composed of only one heavy chain variable region.

As used herein, the term "variable" means that some certain portions of the variable region of an antibody differ in sequence and contribute to the binding and specificity of each particular antibody to its particular antigen. However, the variability is not evenly distributed throughout the antibody variable region. It is concentrated in three fragments in the light and heavy chain variable regions called complementarity determining regions (CDRs) or hypervariable regions. The more conserved portions of the variable regions are referred as framework regions (FRs). The variable regions of the natural heavy and light chains each comprises four FR regions, which are in a substantially β-sheet configuration, and are linked by three CDRs that form a linker ring and, in some cases, form a partial β-sheet structure. The CDRs in each chain stand close together through FR regions and form the antigen-binding site of the antibody together with the CDRs of the other chain (see Kabat et al., NIH Publ. No. 91-3242, Vol. I, 647-669 (1991)). Constant regions are not directly involved in the binding of the antibodies to the antigens, but they exhibit different effector functions, such as involving antibody-dependent cellular cytotoxicity of antibodies.

As known to those skilled in the art, an immunoconjugate and a fusion expression product include: a conjugate formed by connecting a drug, a toxin, a cytokine, a radionuclide, an enzyme and other diagnostic or therapeutic molecules to the antibody or a fragment thereof of the present invention. The present invention also comprises a cell surface marker or antigen binding to the PDL1-targeting nanobody or a fragment thereof.

As used herein, the terms "heavy chain variable region" and "VH" can be used interchangeably.

As used herein, the terms "variable region" and "complementarity determining region (CDR)" can be used interchangeably.

In a preferred embodiment of the present invention, the heavy chain variable region of the antibody comprises three complementarity determining regions, CDR1, CDR2, and CDR3.

In a preferred embodiment of the present invention, the heavy chain of the antibody comprises the above-mentioned heavy chain variable region and a heavy chain constant region.

In the present invention, the terms "the antibody of the present invention", "the protein of the present invention", or "the polypeptide of the present invention" can be used interchangeably and all refer to a polypeptide specifically binding to PDL1, e.g., a protein or polypeptide with a heavy chain variable region. They can contain or do not contain a starting methionine.

The present invention also provides other proteins or fusion expression products comprising the antibody of the present invention. Specifically, the present invention includes any protein or protein conjugate and fusion expression product (i.e., immunoconjugate and fusion expression product) having a heavy chain containing variable regions, as long as the variable region is the same as or has at least 90% homology, preferably at least 95% homology with the variable region of the heavy chain of the antibody of the present invention.

In general, the antigen-binding properties of an antibody can be described by three specific regions located in the heavy chain variable region, referring as variable regions (CDRs), and separated into four framework regions (FRs). The amino acid sequences of four FRs are relatively conservative and do not directly participate in the binding reaction. A cyclic structure is formed by these CDRs that are close to each other in the spatial structure through the β-sheets formed by the FRs between them, and the CDRs on the heavy chains and the CDRs on the corresponding light chains constitute the antigen-binding sites of the antibody. The amino acid sequences of the same type of antibodies can be used to determine which amino acids have constituted the FR or CDR regions.

The variable regions of the heavy chain of the antibody of the present invention are of particular interest because at least part of them involves binding antigens. Therefore, the present invention includes molecules with heavy chain variable regions of antibodies with CDRs, as long as their CDRs have more than 90% (preferably more than 95%, most preferably more than 98%) homology with the CDRs identified here.

The present invention includes not only intact antibodies, but also immunologically active antibody fragments or fusion proteins formed by antibodies and other sequences. Therefore, the present invention also includes fragments, derivatives and analogs of the antibodies.

As used herein, the terms "fragment", "derivative" and "analog" refer to a polypeptide basically maintaining the same biological function or activity of the antibody of the present invention. The polypeptide fragment, derivative or analog of the present invention may be (i) a polypeptide with one or more conservative or non-conservative amino acid residues (preferably the conservative amino acid residues) being substituted, while such substituted amino acid residues may be or may not be encoded by genetic code, or (ii) a polypeptide having substituted group(s) in one or more amino acid residues, or (iii) a polypeptide formed by fusion of the matured polypeptide with another compound (such as the compound that prolongs the half-life of the polypeptide, such as polyethylene glycol), or (iv) a polypeptide formed by fusion of an additional amino acid sequence to the said polypeptide sequence (such as, leader sequence, secretion sequence, or a sequence or a proteinogen sequence used to purify the polypeptide, or a fusion protein formed with a 6His tag). According to the teachings of the present application, these fragments, derivatives, and analogs are within the scope commonly known by those skilled in the art.

The antibody of the present invention refers to a polypeptide having PDL1 protein binding activity and comprising the above-mentioned CDR regions. The term also includes variant forms of polypeptides comprising the CDR regions described above that have the same function as the antibody of the present invention. These variant forms include (but are not limited to): deletion, insertion and/or substitution of one or more amino acids (typically 1-50, preferably 1-30, more preferably 1-20, most preferably 1-10), and addition of one or several amino acids (typically at most 20, preferably at most 10, more preferably at most 5) at the C-terminus and/or N-terminus. For example, in the art, the protein's functions are usually unchanged when an amino acid is substituted by a similar or analogous one. Also, for example, the addition of one or several amino acids at the C-terminus and/or the N-terminus will not normally alter the function of the protein. The term also includes active fragments and active derivatives of the antibody of the present invention.

The variant forms of the antibody include: homologous sequences, conserved variants, allelic variants, natural mutants, induced mutants, proteins encoded by a DNA capable of hybridizing to the coding DNA of the antibody of the present invention under high or low stringency conditions, and a polypeptide or protein obtained using an antiserum against the antibody of the present invention.

The present invention also provides other polypeptides, such as fusion proteins containing antibodies or fragments thereof. In addition to the almost full-length polypeptide, the present invention also includes fragments of the antibody of the present invention. Typically, the fragment has at least about 50 contiguous amino acids, preferably at least about 50 contiguous amino acids, more preferably at least about 80 contiguous amino acids, and most preferably at least about 100 contiguous amino acids of the antibody of the present invention.

In the present invention, "the conservative variant of the antibody of the present invention" refers to a polypeptide that comprises at most 10, preferably at most 8, more preferably at most 5, most preferably at most 3 amino acids replaced by amino acids with the same or similar properties compared with the amino acid sequence of the antibody of the present invention. These conservatively variant polypeptides are preferably produced by amino acid substitution(s) according to Table 2.

**Table 2**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gin |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides a polynucleotide molecule encoding the antibody or a fragment thereof or a fusion protein thereof. The polynucleotides of the present invention can be in a form of DNA or RNA. DNA forms include cDNA, genomic DNA, or synthetic DNA. DNA can be single-stranded or double-stranded. DNA can be the coding strand or the non-coding strand.

The polynucleotides encoding the mature polypeptides of the present invention comprise: coding sequences encoding only the mature polypeptides; coding sequences of the mature polypeptides and various additional coding sequences; coding sequences (and optionally additional coding sequences) of the mature polypeptides, and non-coding sequences.

The term "polynucleotide encoding a polypeptide" may include a polynucleotide that encodes the polypeptide, or a polynucleotide that also includes additional coding and/or non-coding sequences.

The present invention also relates to polynucleotides that hybridize to the sequences as described above and having at least 50%, preferably at least 70%, more preferably at least 80% identity between the two sequences. In particular, the present invention relates to polynucleotides that can hybridize to the polynucleotides of the present invention under stringent conditions. In the present invention, "stringent conditions" means: (1) hybridization and elution at lower ionic strength and higher temperature, such as 0.2 × SSC, 0.1% SDS, 60°C; or (2) hybridization adding with a denaturant, such as 50% (v/v) formamide, 0.1% calf serum/0.1% Ficoll, 42°C, or the like; or (3) hybridization only occurs when the identity between the two sequences is at least 90%, more preferably 95% or more. And the polypeptide encoded by the hybridizable polynucleotide has the same biological function and activity as the mature polypeptide.

The whole length of the nucleotide sequence or the fragment thereof of the antibody of the present invention can be obtained via PCR amplification, recombinant method or artificial synthesis. One feasible method is to synthesize relevant sequences by artificial method, especially when the fragment is short in length. Usually, several small fragments are synthesized first, and then are linked together to obtain a fragment with a long sequence. In addition, the coding sequence of the heavy chain and the expression tag (e.g., 6His) can be fused together to form a fusion protein.

Once a relevant sequence is obtained, the relevant sequence can be obtained in bulk using a recombination method. This is usually carried out by cloning the sequence into a vector, transforming a cell with the vector, and then separating the relevant sequence from the proliferated host cell by conventional methods. The biomolecules (nucleic acids, proteins, etc.) involved in the present invention include biomolecules that exist in an isolated form.

At present, DNA sequences encoding the protein of the present invention (or fragments thereof, or derivatives thereof) can be completely obtained by chemical synthesis. The DNA sequence can then be introduced into a variety of existing DNA molecules (or vectors) and cells known in the art. In addition, mutations can also be introduced into the protein sequences of the present invention by chemical synthesis.

The present invention further relates to a vector comprising said suitable DNA sequence and a suitable promoter or a control sequence. These vectors can be used to transform suitable host cells to enable them to express the protein.

The host cell can be a prokaryotic cell, such as a bacterial cell; or a lower eukaryotic cell, such as a yeast cell; or a higher eukaryotic cell, such as a mammalian cell. Representative examples are: *Escherichia coli*, *streptomactinus*, bacterial cells of *salmonella typhimurium*; fungal cells such as yeast; insect cells of Drosophila S2 or Sf9; animal cells of CHO, COS7, 293 cells, etc.

Transformation of a host cell with a recombinant DNA can be carried out by conventional techniques well known to those skilled in the art. When the host is a prokaryotic organism such as *Escherichia coli*, competent cells that can absorb DNA can be harvested after the exponential growth phase then treated with the CaCl₂ method, and the steps used are well known in the art. Another method is to use MgCl₂. If necessary, the transformation can also be carried out by electroporation. When the host is a eukaryote, the following DNA transfection methods can be selected: calcium phosphate co-precipitation method, conventional mechanical methods such as microinjection, electroporation, liposome packaging, etc.

The obtained transformants can be cultured by conventional methods to express the polypeptide encoded by the gene of the present invention. Depending on the host cell used, the medium used during the culture can be selected from various conventional mediums. The culture is carried out under conditions suitable for the growth of the host cell. When the host cell has grown to an appropriate cell density, a suitable method (such as temperature conversion or chemical induction) is used to induce the selected promoter, and the cell is cultured for another period of time.

The recombinant polypeptide described in the above method can be expressed intracellularly or on the cell membrane, or be secreted out of the cell. If desired, recombinant proteins can be isolated and purified by various separation methods utilizing their physical, chemical, and other properties. These methods are well known to those skilled in the art. Examples of such methods include, but are not limited to: conventional renaturation treatments, treatment with a protein precipitant (salting-out method), centrifugation, osmosis cell disruption, super-treatment, ultracentrifugation, molecular sieve chromatography (gel filtration), adsorption chromatography, ionexchange chromatography, high performance liquid chromatography (HPLC) and various other liquid chromatography techniques and combinations of these methods.

The antibody of the present invention can be used alone, or can be combined or coupled with a detectable label (for diagnostic purposes), a therapeutic agent, a PK (protein kinase) modified moiety, or any combination of these substances.

Detectable labels for diagnostic purposes include, but are not limited to: fluorescent or luminescent labels, radioactive labels, MRI (magnetic resonance imaging) or CT (electronic computer X-ray tomography technique) contrast agents, or enzymes capable of producing detectable products.

A therapeutic agent that can be combined or coupled with the antibody of the present invention includes, but is not limited: 1. a radionuclide; 2. a biological toxin; 3. a cytokine such as IL-2, etc.; 4. a gold nanoparticle/nanorod; 5. a viral particle; 6. a liposome; 7. a magnetic nanoparticle; 8. a prodrug-activating enzyme (e.g., DT-diaphorase (DTD) or biphenyl hydrolase-like protein (BPHL)), etc.

### PDL1 protein

Programmed cell death ligand 1 (PDL1), also known as Cluster of differentiation 274 (CD274) or B7 homolog 1 (B7-H1), is a protein in the human body encoded by the CD274 gene. PDL1 is a type 1 transmembrane protein with a size of 40 kDa, believed to be associated with immune system suppression in certain special situations (such as pregnancy, tissue transplantation, autoimmune diseases, and certain diseases such as hepatitis). Under normal circumstances, the immune system responds to foreign antigens that accumulate in lymph nodes or spleen, promoting antigen-specific cytotoxic T cell (CD8+ T cell proliferation). The binding of programmed cell death receptor-1 (PD-1) and programmed cell death ligand 1 (PDL1) can transmit inhibitory signals, reduce the proliferation of CD8+ T cells in lymph nodes. PD-1 can also regulate the Bcl-2 gene to control the accumulation of antigen-specific T cells in lymph nodes.

### PD-1/PDL1

The binding of PDL 1 and PD-1, as an inhibitory signal for mediating T cell activation, inhibits the killing function of T cells and has a negative regulatory effect on the human immune response.

### Pharmaceutical composition

The present invention further provides a composition. Preferably, the composition is a pharmaceutical composition comprising the antibody, or an active fragment or a fusion protein thereof, and a pharmaceutically acceptable carrier. In general, these substances may be formulated in a non-toxic, inert and pharmaceutically acceptable aqueous carrier medium, wherein the pH is generally about 5-8, preferably, pH is about 6-8, though the pH value may be varied depending on the nature of the substances to be formulated and the condition to be treated. The formulated pharmaceutical composition may be administered by conventional routes, including (but not limited to): intraperitoneal, intravenous, or topical administration.

The pharmaceutical composition of the present invention comprises a safe and effective amount (e.g., 0.001-99 wt %, preferably 0.01-90 wt %, more preferably 0.1-80 wt %) of the antibody (or a conjugate thereof) of the present invention and a pharmaceutically acceptable carrier or excipient. Such carriers include (but are not limited to): saline, buffer, glucose, water, glycerol, ethanol, and a combination thereof. Pharmaceutical preparations should correspond to the administration modes. The pharmaceutical composition of the present invention can be prepared in the form of an injection, for example, can be prepared by a conventional method using physiological saline or an aqueous solution containing glucose and other adjuvants. The pharmaceutical composition, for example, an injection or a solution, should be prepared under aseptic conditions. The administration amount of an active ingredient is a therapeutically effective amount, for example, about 10 µg per kilogram of body weight to about 50 mg per kilogram of body weight daily. In addition, the polypeptide of the present invention may also be used in combination with other therapeutic agents.

When the pharmaceutical composition is used, a safe and effective amount of immunoconjugate is administered to a mammal, wherein the safe and effective amount is generally at least about 10 µg per kilogram of body weight, and in most cases, no more than about 50 mg per kilogram of body weight, preferably, the amount is from about 10 µg per kilogram of body weight to about 10 mg per kilogram of body weight. Of course, a specific amount should also depend on the factors such as administration route and physical conditions of a patient, which are all within the skills of skilled physicians.

### PDL1-targeting nanobody

In the present invention, the PDL1-targeting nanobody includes a monomer, bivalent (bivalent antibody), tetravalent (tetravalent antibody), and/or multivalent (multivalent antibody).

In one preferred embodiment of the present invention, the PDL1-targeting nanobody comprises one or more of the VHH chains having the amino acid sequence as shown in SEQ ID NO: 1, SEQ ID NO: 2, SEQ ID NO: 3, SEQ ID NO: 4, SEQ ID NO: 5, or SEQ ID NO: 6.

### Labeled antibody

In one preferred embodiment of the present invention, the antibody carries a detectable label. Preferably, the label is selected from the group consisting of: an isotope, a colloidal gold label, a colored label, or a fluorescent label.

Colloidal gold labeling can be carried out using methods known to those skilled in the art. In one preferred embodiment of the present invention, the PDL1 protein-targeting antibody is labeled with colloidal gold to obtain a colloidal gold labeled antibody.

The PDL1-targeting nanobody of the present invention can effectively bind to PDL1 protein.

### Detection method

The present invention also relates to a method for detecting PDL1 protein or a fragment thereof. The steps of the method are roughly as follows: obtaining a cell and/or tissue sample; dissolving the sample in a medium; and detecting the level of PDL1 protein in the dissolved sample.

In the detection method of the present invention, the sample used is not particularly limited, and a representative example is a cell-containing sample present in a cell preservation solution.

### Kit

The present invention also provides a kit comprising the antibody (or a fragment thereof) or the detection plate of the present invention. In one preferred embodiment of the present invention, the kit further comprises a container, instructions, and a buffer, etc.

The present invention also provides a detection kit for detecting the PDL1 protein level, which comprises an antibody that recognizes PDL1 proteins, a lysis medium for dissolving a sample, common reagents and buffers required for detection, such as various buffers, detection labels, detection substrates, etc. The detection kit may be an *in vitro* diagnostic device.

### Application

As described above, the antibody of the present invention has a wide range of biological application value and clinical application value, and its application relates to the diagnosis and treatment, basic medical research, biological research and other fields of the PDL1 protein-related diseases. One preferred application is for clinical diagnosis, prevention and treatment targeting PDL1 protein.

The present invention also provides a method for stimulating an immune response mediated by T cells targeting mammalian tumor cell populations or tissues, which comprises the following step of: administering the CAR-T cell of the present invention to a mammal.

In one embodiment, the present invention comprises a type of cell therapy, in which autologous T cells are isolated from the patient (or a heterologous donor), then activated and genetically modified to produce CAR-T cells, and the CAR-T cells are then injected into the same patient's body. This approach extremely reduces the probability of graft-versus-host reactions, and the antigen is recognized by T cells in an MHC-free manner. In addition, one kind of CAR-T can treat all cancers that express this antigen. Unlike antibody therapy, CAR-T cells can replicate *in vivo*, producing long-term persistence that can lead to sustained control of tumors.

In one embodiment, the CAR-T cells of the present invention can undergo stable *in vivo* amplification and can last for several months to years. In addition, CAR-mediated immune response can be part of the adoptive immunotherapy step, wherein CAR-T cells can induce a specific immune response against tumor cells with high expression of antigens recognized by the CAR antigen-binding domains. For example, the CAR-T cell of the present invention induces a specific immune response against tumor cells with high expression of PDL 1.

Treatable cancers include tumors that have not been vascularized or substantially have not been vascularized, as well as vascularized tumors. Cancer types treated by the CAR of the present invention include but are not limited to: gastric cancer, lung cancer, liver cancer, osteosarcoma, breast cancer, pancreatic cancer, lymphoma, etc.

Typically, activated and expanded cells as described herein can be used for the treatment and prevention of diseases such as tumors. Therefore, the present invention provides a method for treating cancer, which comprises administering an therapeutically effective amount of the CAR-T cell of the present invention, to a subject in need thereof.

The CAR-T cell of the present invention can be administered alone or as a pharmaceutical composition with diluents and/or other components such as IL-2, IL-17, or other cytokines or cell populations. Anyway, the pharmaceutical composition of the present invention may include a target cell population as described herein, combined with one or more pharmaceutically or physiologically acceptable carriers, diluents, or excipients.

The pharmaceutical composition of the present invention can be administered in a manner suitable for the disease to be treated (or prevented). The quantity and frequency of administration will be determined by factors such as the patient's condition, the type and severity of the patient's disease, or can be determined by clinical trials.

When refer to "immunologically effective amount", "antitumor effective amount", "tumor-inhibiting effective amount" or "therapeutic amount", the precise amount of the composition of the present invention to be administered can be determined by a physician, taking into account the age, weight, tumor size, degree of infection or metastasis, and individual differences in the condition of the patient (subject). The pharmaceutical composition comprising the T cell described herein may be administered in a dose of 10⁴ to 10⁹ cells per kg of body weight, preferably a dose of 10⁵ to 10⁷ cells per kg of body weight (including all integer values within the range). The T-cell composition can also be administered multiple times at these doses. The cells can be administered using injection techniques well known in immunotherapy (see, for example, Rosenberg et al., New Eng. J. of Med. 319:1676, 1988). The optimal dose and treatment plan for a specific patient can be easily determined by those skilled in the field of medicine by monitoring the patient's disease signs and adjusting the treatment accordingly.

The administration of the composition to the subject can be carried out in any convenient manner, including by spray method, injection, swallowing, infusion, implantation, or transplantation. The composition described herein can be administered to the patient subcutaneously, intradermally, intratumorally, intranodally, intramedullary, intramuscularly, intravenously, or intraperitoneally. In one embodiment, the T-cell composition of the present invention is administered to the patient by intradermal or subcutaneous injection. In another embodiment, the T-cell composition of the present invention is preferably administered by intravenous injection. The composition of T cells can be directly injected into a tumor, lymph node, or infection site.

In certain embodiments of the present invention, the cells activated and expanded using the methods described herein or other methods known in the field to expand T cells to therapeutic levels are administered to the patient in combination with (e.g., before, simultaneously, or after) any number of related therapeutic modalities, including but not limited to treatments with the following agents: such as antiviral therapies with cidofovir and interleukin-2, cytosine arabinoside (also known as ARA-C), or natalizumab treatment for MS patients, or efalizumab treatment for psoriasis patients, or other treatments for PML patients. In further embodiments, the T cell of the present invention can be used in combination with the following: chemotherapy, radiation, immunosuppressants such as cyclosporine, azathioprine, methotrexate, mycophenolate mofetil and FK506, antibodies, or other immunotherapeutic agents. In further embodiments, the cellular composition of the present invention is administered to the patient in combination with (e.g., before, simultaneously, or after) bone marrow transplantation, chemotherapy agents such as fludarabine, external beam radiation therapy (XRT), and cyclophosphamide. For example, in one embodiment, the subject may undergo a standard treatment of high-dose chemotherapy followed by peripheral blood stem cell transplantation. In some embodiments, after transplantation, the subject receives an infusion of the expanded immune cells of the present invention. In an additional embodiment, the expanded cells are administered before or after surgical procedures.

The dosage of the above treatments administered to the patient will vary depending on the precise nature of the condition being treated and the recipient of the treatment. Dosages for human administration can be administered according to accepted practices in the field. Typically, from 1×10⁵ to 1×10¹⁰ modified T cells of the present invention may be administered to a patient via for example, intravenous infusion per treatment or per course of treatment.

### Reagents, consumables and equipment

### Main reagents

Agar (Sigma, CAT# A1296), Peptone (Sigma, CAT#93926), Yeast Extract (OXOID, CAT#: LP0021), Sodium Chloride (Aladdin, CAT#: C111533), Potassium Chloride (Aladdin, CAT#: P112133), Magnesium Sulfate (Sinopharm, CAT#: 10013018), Magnesium Chloride (Sinopharm, CAT#: 10012818), Glucose (Sangon, CAT#: GT1991); SfiI (NEB, CAT#: R0123L), T4 DNA ligase (TaKaRa, CAT#: 2011A), PrimeScript^{™} II 1st Strand cDNA Synthesis Kit (TaKaRa, CAT#: 6210B), NuHi power mix (NUHIGH Bio, CAT#: NH9303), 3M Sodium Acetate (pH 5.2-6) (Sigma, CAT#: 126-96-5), DNA Fragment Recovery Kit (TakaRa, CAT#: 9761), Gel Recovery Kit (Qiagen, CAT#: 28706), Tiangen Plasmid Extraction Kit (Tiangen, CAT#: DP117), HRP-M13 (Sino Biolo, CAT#: 11973-MM05), PE-anti-Human IgG (eBioscience, Cat#: 12-4998-82), Rabbit anti-Llama IgG (H+L) Secondary Antibody [HRP] (Novus, CAT# NBP1-75095), SS320 Competent Cells (iCarTab), pComF Phage Display Vector (iCarTab), NHS-biotin (APExBIO, CAT#: A8002), HRP-Streptavidin (Boster, CAT#: BA1088), Streptomycin avidin magnetic beads (NEB, CAT #: S14205), Antibody affinity testing buffer: HBS-EP+10X (GE, Cat # BR100669), Amino coupling reagent kit (GE, Cat # BR100050), 10 mM Glycine 2.5 (GE, Cat#BR100356), S-series CM5 chip (GE, Cat # 29149603), PBS (Gbico, CAT# 14190-250), DMEM (Gbico, CAT# 41965-062), RPMI1640 (Gbico, CAT# 61870044), FBS (Gbico, CAT# 10099-141), Genomic DNA Purification Kit (Lifetech, CAT#K0512), Polybrene (Sigma, CAT#107689-10G), LVtransm transfection reagent (iCarTab, Cat # LVTran100), Lymphocyte isolation solution (Stem Cell, CAT # 18051).

### Main consumables

50 mL Falcon Tubes (Corning, CAT# 352070), Electroporation Cuvettes (Bio-Rad 0.2 cm), RNase free 1.5 mL EP Tubes (QSP, CAT#: 509-GRD-Q), 200 µL RNase free PCR Tubes (Axygen, PCR-02D-C), T125 Flask (Corning, CAT# 431143), 15 mL Falcon Tubes (Corning, CAT# 430052), 6-well plate (Corning, CAT# 3516), 96-well plate (Corning, CAT# 3365).

### Main equipments

Electroporator (Eppendorf Multiporator), Centrifuge (Xiangyi, H1650R), Constant Temperature Incubator (Shanghai Jinghong, DNP-9052), Constant Temperature Shaking Incubator (Langyue, DZ-85A), Super Clean Bench (Sujing Antai, SW-CJ-1FD), PCR (Applied Biosystems, ABI2720), Biological Safety Cabinet (Haier, HR40-IIA2), Flow Cytometer (Thermo Attune Nxt flow cytometer), Thermo 3111 CO₂ Incubator, BiaCore T200.

### Amino acid sequences

SEQ ID NO: 1 (Nanobody 17-A06) 
MAQVKLEESGGGLVQAGGSLRLSCVASGRSFITYAVGWFRQAPGKEREFVASINWSGAMTYYTD
SVNGRFAISRDNAKNTVYLQMNNLKLEDTAVYYCASTISAVTPTNGYQNWGQGTQVTVSS
SEQ ID NO: 2 (Nanobody 17-C12) 
MAQVKLEESGGGLVQPGGSLTLSCAASGRTFGFYGWFRQAPGKEREFVAGITWGGSVTSYADSV
KGRFTISRDNAKNTVYLQMNSLKPENTAVYYCARSPRVTTTPREFDVWGQGTQVTVSS
SEQ ID NO: 3 (Nanobody 17-A01) MAAVQLVDSGGGLVQPGGSLRLSCAASGRTFGFYGWFRQAPGKEREFVAAITWGGSAISYEDSA KGRFTISRDNAKNTVYLQMNSLKPEDTAVYYCARSTRVTTNPREYDYWGQGTQVTVSS
SEQ ID NO: 4 (Nanobody 17-G06) 
MAAVQLVESGGGLVQPGGSLRLSCAASGRTFGFYGWFRQAPGKEREFVAAITWGGSAISYDDSA
KGRFTISRDNAKNTVYLQMNSLKPEDTAVYYCARSMRVTTNPREYDYWGQGTQVTVSS
SEQ ID NO: 5 (Nanobody 23-G3) 
MAAVQLVESGGGLVQAGGSLRLSCVASGRSFITYAVGWFRQAPGKEREFVASVNWSGAMTYYA
DAVNGRFTISRDNAKNTVYLQMNNLKLEDTAVYYCAATISAVTPTNGYQNWGQGTQVTVSS
SEQ ID NO: 6 (Nanobody 29-H9) 
RLQLVESGGGLVQAGGSLRLSCAASGFTFSTYAMSWYRGVPEKERELVAFISSDGGGTTYRDSVK
GRFTISRDNGKNTVYLQMNSLKPEDTGVYYCARGLAQIWGQGTQVTVSS
SEQ ID NO: 7 (CDR1 of nanobodies 17-A06, 23-G3)
   GRSFITYA
SEQ ID NO: 8 (CDR2 of nanobody 17-A06)
   INWSGAMT
SEQ ID NO: 9 (CDR3 of nanobody 17-A06)
   ASTISAVTPTNGYQN
SEQ ID NO: 10 (CDR1 of nanobodies 17-C12, 17-A01, 17-G06)
   GRTFGF
SEQ ID NO: 11 (CDR2 of nanobody 17-C12)
   ITWGGSVT
SEQ ID NO: 12 (CDR3 of nanobody 17-C12)
   ARSPRVTTTPREFDV
SEQ ID NO: 13 (CDR2 of nanobodies 17-A01, 17-G06)
   ITWGGSAI
SEQ ID NO: 14 (CDR3 of nanobody 17-A01)
   ARSTRVTTNPREYDY
SEQ ID NO: 15 (CDR3 of nanobody 17-G06)
   ARSMRVTTNPREYDY
SEQ ID NO: 16 (CDR2 of nanobody 23-G3)
   VNW SGAMT
SEQ ID NO: 17 (CDR3 of nanobody 23-G3)
   AATISAVTPTNGYQN
SEQ ID NO: 18 (CDR1 of nanobody 29-H9)
   GFTFSTYA
SEQ ID NO: 19 (CDR2 of nanobody 29-H9)
   ISSDGGGT
SEQ ID NO: 20 (CDR3 of nanobody 29-H9)
   ARGLAQI
SEQ ID NO: 21 (FR1 of nanobody 17-A06)
   MAQVKLEESGGGLVQAGGSLRLSCVAS
SEQ ID NO: 22 (FR2 of nanobodies 17-A06, 23-G3)
   VGWFRQAPGKEREFVAS
SEQ ID NO: 23 (FR3 of nanobody 17-A06)
   YYTDSVNGRFAISRDNAKNTVYLQMNNLKLEDTAVYYC
SEQ ID NO: 24 (FR4 of nanobodies 17-A06, 17-C12, 17-A01, 17-G06, 23-G3, 29-H9)
   WGQGTQVTVSS
SEQ ID NO: 25 (FR1 of nanobody 17-C12)
   MAQVKLEESGGGLVQPGGSLTLSCAAS
SEQ ID NO: 26 (FR2 of nanobody 17-C12)
   YGWFRQAPGKEREFVAG
SEQ ID NO: 27 (FR3 of nanobody 17-C12)
   SYADSVKGRFTISRDNAKNTVYLQMNSLKPENTAVYYC
SEQ ID NO: 28 (FR1 of nanobody 17-A01)
   MAAVQLVDSGGGLVQPGGSLRLSCAAS
SEQ ID NO: 29 (FR2 of nanobodies 17-A01, 17-G06)
   YGWFRQAPGKEREFVAA
SEQ ID NO: 30 (FR3 of nanobody 17-A01)
   SYEDSAKGRFTISRDNAKNTVYLQMNSLKPEDTAVYYC
SEQ ID NO: 31 (FR1 of nanobody 17-G06)
   MAAVQLVESGGGLVQPGGSLRLSCAAS
SEQ ID NO: 32 (FR3 of nanobody 17-G06)
   SYDDSAKGRFTISRDNAKNTVYLQMNSLKPEDTAVYYC
SEQ ID NO: 33 (FR1 of nanobody 23-G3)
   MAAVQLVESGGGLVQAGGSLRLSCVAS
SEQ ID NO: 34 (FR3 of nanobody 23-G3)
   YYADAVNGRFTISRDNAKNTVYLQMNNLKLEDTAVYYC
SEQ ID NO: 35 (FR1 of nanobody 29-H9)
   RLQLVESGGGLVQAGGSLRLSCAAS
SEQ ID NO: 36 (FR2 of nanobody 29-H9)
   MSWYRGVPEKERELVAF
SEQ ID NO: 37 (FR3 of nanobody 29-H9)
   TYRDSVKGRFTISRDNGKNTVYLQMNSLKPEDTGVYYC

### Main advantages of the present invention include:

1. The PDL1 nanobodies of the present invention can specifically bind to PDL1 and have high affinity.
2. The PDL1 nanobodies of the present invention have good PD-1-PDL1 blocking activity and high neutralizing activity against PDL1.
3. Both monovalent and divalent PDL1 nanobodies are constructed in the present invention, and they both exhibit significant blocking activity.
4. The PDL1 nanobodies of the present invention only block the PD-1 and PDL1 pathway and do not affect the PD-1 and PD-L2 pathway, thereby avoiding the occurrence of side effects such as interstitial pneumonia and improving safety.
5. The PDL1 nanobodies of the present invention can more comprehensively activate the immune system to kill tumors, and can significantly benefit patients for whom PD-1 therapy is ineffective or who are resistant to PD-1 therapy, and achieve therapeutic effects. Therefore, the scope of patients suitable for treatment is broadened.
6. The PDL1 nanobodies of the present invention can not only inhibit the PD-1-PDL1 pathway, but also facilitate the comprehensive activation of T cell function and cytokine production by blocking the co-inhibitory function of B7.1 and PDL1, thereby achieving better immune effects.
7. The PDL1 nanobodies of the present invention are easier to produce in bulk at a lower cost compared to polyclonal antibodies, which can reduce production costs and thus lower the price of drugs prepared from them.
8. The PDL1 nanobodies of the present invention have a wide range of applicable conditions: they are stable over a wider temperature range and can still function at high temperatures. Unlike traditional antibody fragments, the unfolding of nanobodies at high temperatures has been proven to be completely reversible. In addition, nanobodies are also stable at extreme pH values and can survive in succus gastricus.
9. In terms of structure, due to the hydrophilic side of the nanobodies, the PDL1 nanobodies of the present invention do not have solubility issues and aggregation issues related to traditional antibodies.

The present invention is further illustrated below in conjunction with specific examples. It should be understood that these examples are only used to illustrate the present invention and not to limit the scope of the present invention. The experimental methods in the following examples which do not specify the specific conditions are usually in accordance with conventional conditions, such as conditions described in Sambrook et al., Molecular Cloning: A Laboratory Manual (New York: Cold Spring Harbor Laboratory Press, 1989), or in accordance with the conditions recommended by the manufacturer. Unless otherwise stated, percentages and parts are calculated by weight.

### Example 1. Antigen preparation scheme

1. Sequence of the extracellular domain of PDL1 (19AA-238AA) was generated by gene synthesis, followed by attachment of an IgG1 Fc tag at the C-terminus. The construct was subcloned into a eukaryotic expression vector to form the antigen PDL1-Fc protein expression vector.
2) The prepared PDL1-Fc protein expression vectors were subjected to large-scale plasmid extraction, then transfected into 293 cells. Cells were continuously cultured for 8 days and centrifugated to collect the supernatant. The supernatant was filtered through a 0.45 µm filter membrane, and the filtrate was transferred to a sterile centrifuge tube. The PDL1-Fc protein was obtained by purification using a protein A column.
3. The PDL1-Fc protein was identified using SDS-PAGE detection.

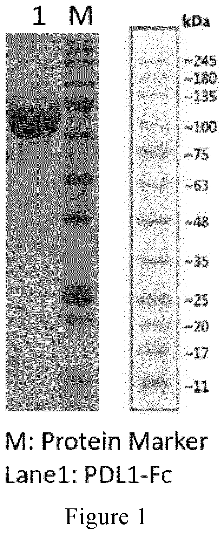

As shown in Figure 1, SDS-PAGE detection shows that the actual SDS-PAGE molecular weight of PDL1-Fc is about 65 kD, which is consistent with the electrophoresis detection results of ACRO's PDL1-Fc product. Moreover, the purity of the PDL1-Fc band of the present invention is high, indicating good expression and purification effects.

The purified PDL1-Fc protein is an antigen, also known as an immunogen or immune antigen.

### Example 2. Antigen immunization of llamas

The above prepared antigen was used to immunize a llama through subcutaneous multi-point injection. The process of three immunizations is shown in Table 3.

**Table 3**

| | Llama Immunization Schedule |
|---|---|
| Primary immunization | 5 mL blood was collected as negative serum before immunization; the adjuvant was mixed with the antigen (1 mg) at 1:1, emulsified, and subcutaneously injected at multiple points. |
| Second immunization | Antigen (0.5 mg) was mixed with the adjuvant at 1:1, emulsified, and subcutaneously injected at multiple points. |
| Third immunization | Antigen (0.5 mg) was mixed with the adjuvant at 1:1, emulsified, and subcutaneously injected at multiple points. |
| | Peripheral blood was collected 10 days after the third immunization, and serum was prepared for ELISA titer testing. If the serum meets the requirements after the third immunization, indicating successful immunization, 100 mL of peripheral blood was collected for the construction of a phage display library. |

### Example 3. Detection of immunological potency

1. 5 mL of peripheral blood after the third immunization in Example 1 was collected, and the centrifuge tube containing the blood sample was placed in a 37°C incubator for 1 hour. Then, the blood sample was transferred to 4°C overnight.
2. The serum was transferred to a new sterile centrifuge tube and centrifuged at 5000 rpm for 20 minutes. A 96-well plate pre-coated with PDL1 (Fc-cut) antigen was used, and anti-camel IgG (H+L) secondary antibody and anti-camel VHH secondary antibody were selected for ELISA experiments to detect the immune titer.

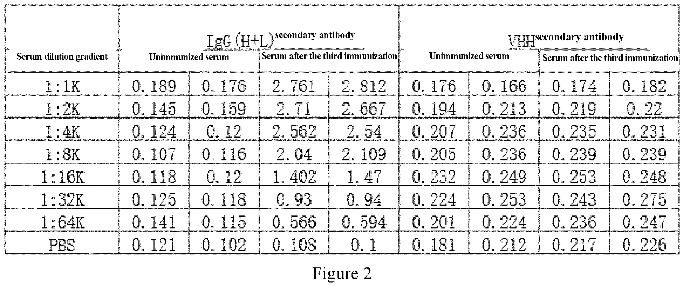

As shown in the ELISA test results in Figure 2, compared with the unimmunized (negative) serum, when using anti-camel IgG (H+L) secondary antibody for detection, the immune serum was positive and had a high titer.

These results indicate that the immune serum specifically binds to PDL1, and the OD450 value changes accordingly with the serum dilution gradient, indicating successful immunization.

100 mL of peripheral blood was collected and arranged for library construction.

### Example 4. PBMC isolation and obtaining of VHH antibody fragments

1. 150 mL of peripheral blood prepared in Example 1 was collected, and PBMCs were isolated using lymphocyte separation medium.
2. RNA was extracted and subjected to reverse transcription using the PrimeScript^{™} II 1st Strand cDNA Synthesis Kit to prepare cDNA.
(1) Reaction mixture Mix1 shown in Table 4 was prepared in a 200 µL PCR tube:

**Table 4**

| Reagent | Quantity |
|---|---|
| OligodT Primer (50 µM) | 8 µL |
| dNTP Mixture (10 mM each) | 8 µL |
| Total RNA sample | 20 µg |
| RNase-Free Water | up to 80 µL |

(2) After incubation at 65°C for 5 minutes, the mixture was rapidly cooled on ice.
(3) The reaction solution shown in Table 5 was prepared in the same PCR tube as described above:

**Table 5**

| Reagent | Quantity |
|---|---|
| The above denatured reaction mixture | 80 µL |
| 5×PrimeScript II Buffer | 32 µL |
| RNase Inhibitor (40 M/µL) | 4 µL |
| PrimeScript II RTase (200 M/µL) | 8 µL |
| RNase-Free Water 36 | µL |

(4) After thorough mixing, 80 µL of the solution was dispensed into each tube and placed in a PCR instrument at 42°C for 1 hour, followed by heat-inactivation at 70°C for 15 minutes. Finally, the cDNA samples were placed on ice or stored at -20°C for long-term preservation.
3. Amplification of VHH fragments
(1) The first round PCR reaction system (50 µL/tube) was prepared according to Table 6:

**Table 6**

| Component | Quantity |
|---|---|
| Upstream Primer (5 µM) | 2 µL |
| Downstream Primer (10 µM) | 1 µL |
| NuHi Power mix (2×) | 25 µL |
| cDNA Template | 2 µL |
| Sterile Water | 20 µL |

After the preparation of PCR reaction system, the PCR instrument was set up according to the procedure shown in Table 7:

**Table 7**

| Program | Temperature | Time | Number of cycles |
|---|---|---|---|
| Pre-denaturation | 95°C | 10 min | |
| Denaturation | 95°C | 15 s | 30× |
| Annealing | 55°C | 30 s | |
| Extension | 68°C | 1 min | |
| Final extension | 68°C | 10 min | |

(2) Agarose electrophoresis of PCR products:
Electrophoresis analysis on PCR products was performed by using 1% agarose, and fragments with a molecular weight of approximately 750 bp were isolated. PCR products were recovered by a gel recovery kit and subjected to the determination of concentration by NanoDrop.
(3) The second round PCR reaction system (50 µL/tube) was prepared according to Table 8.

**Table 8**

| Component | Quantity |
|---|---|
| 2^{nd} F-Primer | 2 µL |
| 2^{nd} R-Primer | 2 µL |
| NuHi Power mix (2×) | 25 µL |
| Products recovered from the first round PCR | 200 ng |
| Sterile Water | Add to 50 µL |

After the preparation of PCR reaction system, the PCR instrument was set up according to the procedure in Table 9:

**Table 9**

| Program | Temperature | Time | Number of cycles |
|---|---|---|---|
| Pre-denaturation | 95°C | 10 min | |
| Denaturation | 95°C | 15 s | 25× |
| Annealing | 55°C | 30 s | |
| Extension | 68°C | 1 min | |
| Final extension | 68°C | 10 min | |

(4) Agarose electrophoresis analysis of the second round PCR products:
Electrophoresis analysis on PCR products was performed by using 1% agarose, and VHH fragments with a molecular weight of approximately 400 bp were isolated. VHH PCR products were recovered by a gel recovery kit and subjected to the determination of concentration by NanoDrop.

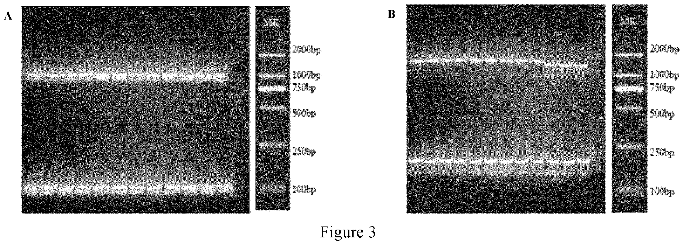

Figure 3 shows the results of agarose gel electrophoresis of PCR products: Figure 3A shows that two PCR bands of about 1000 bp and 750 bp were obtained in the first round of PCR, and 750 bp fragments were recovered from the gel as templates for the second round of PCR; Figure 3B shows that the second round of PCR obtained about 450 bp bands, which were identified as VHH fragments by agarose gel electrophoresis.

The VHH fragments were cleaved with SfiI enzyme and subcloned into the phage display vector pComF. The ligation products were then electroconverted into SS320 *E. coli* competent cells to construct a single domain antibody phage display library.

### Example 5. Construction of phage display library

1. Construction of phage display vector:
   (1) The pCom F vector and the recovered VHH PCR gel product obtained in Example 4 were digested with SfiI at 50°C overnight, respectively.
   (2) The pCom F vector fragments were isolated using 1% agarose gel. The gel of 5000 bp vector fragments was excised and recovered. Meanwhile, the PCR-digested product was purified using DNA Fragment Recovery Kit, and subjected to the determination of concentration by using NanoDrop.
   (3) The digested pCom F vector and VHH fragment were ligated using T4 ligase at 16°C overnight.
2. Electrotransformation of phage ligation product into *E. coli*:
   (1) Electroporation cuvettes, ligation product, and electrotransformation competent cells were placed on ice for pre-cooling;
   (2) The pre-cooled library ligation product was added to the electrotransformation competent cells, kept on ice for 1 minute, and 70 µL of DNA/competent cell mixture was added to each electroporation cuvette, which was then placed on ice;
   (3) Electrotransformation was performed at 2500 V for 5 ms;
   (4) After electroporation, cells were immediately resuspended by adding SOC culture medium equilibrated to room temperature, and cultured in a shaker at 37 °C for 1 hour;
   (5) 15 mL of the bacterial suspension was directly used for phage rescue, while the remaining 5 mL of the electroporation product was mixed with an equal volume of 50% glycerol, thoroughly mixed, and stored at -80°C;
   (6) Additionally, 20 µL of the bacterial suspension was diluted in 980 µL of 2YT culture medium, and 100 µL of the diluted product was further diluted in 900 µL of 2YT culture medium. 50 µL of the secondarily diluted product was evenly spread on a LB plate containing ampicillin and cultured at 37°C overnight.
   (7) The next day, the plate was taken out to count the number of clones produced by each ligation and calculate the library capacity;
   (8) Simultaneously, 20 monoclonal colonies from the plate were picked and cultured in 2YT culture medium containing ampicillin at 37°C for approximately 6-8 hours. The bacterial suspension was then subjected to sequencing (by using universal primer M13R), in order to determine the diversity of the library.

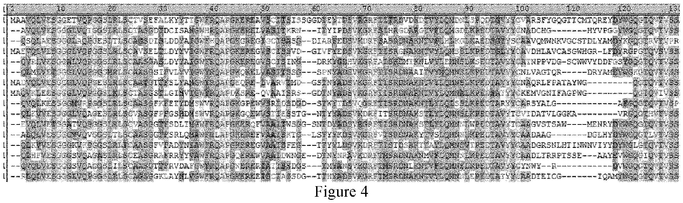

As shown in Figure 4, the alignment results of the phage display library sequences show that the empty-vector rate and antibody repeat rate of the phage display library were satisfactory, and the *Escherichia coli* library capacity was 6.53E8.

### Example 6. Recovery and rescue of phage display library and phage precipitation

1. The electrotransformation product after electrotransformation in Example 5 was diluted with 2YT to adjust the OD600 to approximately 0.2. Ampicillin with a final concentration of 100 µg/mL was added, and the mixture was incubated in a constant temperature shaker at 37°C and 225 rpm until the OD600 reached 0.5.
2. M13KO7 was added. After thorough mixing, the mixture was allowed to stand at 37°C for 30 minutes and then cultured at 37°C and 225 rpm for 1 hour.
3. Volume of M13KO7 = 10 × Volume × OD600 × 5 × 10⁸ / Titer of M13KO7.
4. The bacterial suspension was centrifuged at 6000 rpm for 10 minutes and resuspended in 2YT-AK medium. It was then cultured overnight at 25°C and 200 rpm.
5. The bacterial suspension was centrifuged at 10000 rpm for 15 minutes.
6. The precipitate was discarded, and the supernatant was transferred to a new centrifuge tube. PEG/NaCl with a volume of 1/5 bacterial suspension was added to the tube, mixed thoroughly, and the mixture was placed at 4°C for 2 hours.
7. The precipitated phage supernatant was centrifuged at 10000 rpm, 4°C, for 30 minutes. The supernatant was discarded, and the precipitate (phage) in each 50 mL centrifuge tube was resuspended in 1 mL of sterile PBS.
8. The resuspended phage was transferred to a 1.5 mL EP tube and centrifuged at 12000 g, 4°C, for 5 minutes.
9. The supernatant was transferred to a new 1.5 mL EP tube, and 250 µL of PEG/NaCl was added to each tube. After thorough mixing, the tubes were placed at 4°C for 10 minutes.
10. Centrifugation was performed at 12000g for 10 minutes. The supernatant was discarded, and 1 mL of PBS was added for resuspension.
11. Centrifugation was performed at 12000g for 5 minutes. The precipitate was discarded, and the supernatant was transferred to a new 1.5 mL EP tube.
12. Centrifugation was performed at 12000g for 5 minutes, and the supernatant was transferred to a new 1.5 mL EP tube, thereby obtaining the original phage library.
13. 10 µL of the precipitate was added to 90 µL of 2YT medium, labeled as 10⁻¹, and serially 10-fold diluted to 10⁻⁹. 20 µL of the gradient diluted samples from the 10⁻⁷, 10⁻⁸, and 10⁻⁹ were added to 200 µL of pre-prepared ER2738 with an OD600 of 0.5. The mixture was thoroughly mixed and placed in a 37°C water bath for 10 minutes. Each 108 µL of the mixture was spread onto an LB-AMP solid plate and incubated overnight at 37°C. The number of plaques was counted the next day to determine the titer.
14. Calculation of titer: Plates with a plaque number between 30-300 were selected. Titer was obtained by taking the average of two plates, multiplying the plaque number by the dilution fold and then multiplying by 100.

The phage display library that has undergone recovery, rescue, and counting can be used for subsequent phage display library solutionpanning.

### Example 7. Solutionpanning of phage display library

Utilizing Streptavidin magnetic beads, biotinylated target antigens were pre-bound and then incubated with the phage library to be screened. Non-specifically bound phages were washed away, and the recombinant phages bound to the target antigens were eluted with TEA for amplification. After 3-4 rounds of panning, monoclonal clones were selected for sequencing.
1. The biotinylated target antigens were diluted with PBS to concentrations of 50, 10, 5, and 5 µg/ml (varying concentrations for each round) and mixed with streptavidin magnetic beads for 1 hour of incubation at 4°C. The unbound target antigens were washed away.
2. Approximately 6×10¹¹ Pfu of 150 µL lib phage or the amplification product from the previous round was diluted to 600 µL and added to ELISA wells pre-coated with Fc for Fc depletion, and the Fc depletion was performed in three rounds to remove Fc-bound phages.
3. The phages after Fc depletion were mixed with streptavidin magnetic beads and incubated at 4°C for 1 hour to remove streptavidin-bound phages.
4. The magnetic beads were discarded, and the phages were diluted to approximately 1 mL with 1% PBSA. They were then mixed with the magnetic beads pre-bound with the target antigens from Step 1 and incubated at 4°C for 1 hour.
5. The phages were aspirated, and the magnetic beads were washed 3-5 times with PBS for 2-3 minutes each. At the same time, 1.5 mL EP tubes previously blocked with MPBS were also washed with PBS.
6. The phages bound to the magnetic beads were eluted with 600 µL of 1×TEA for 10 minutes. The eluted product was transferred to a pre-blocked EP tube and neutralized with 300 µL of Tris-HCl.
7. 10 µL of the output product was added to 90 µL of 2YT medium, labeled as 100, and then sequentially diluted 10-fold to 10⁻². 20 µL of diluted samples from the 10¹, 10⁰, 10⁻¹, and 10⁻² gradients were added to 200 µL of pre-prepared ER2738 with an OD600 of 0.5 (10¹ was the undiluted product directly added to ER2738). After mixing, the mixture was placed in a 37°C water bath for 10 minutes, and each 108 µL was spread onto an LB-AMP solid plate. The plates were incubated at 37°C overnight, and the number of plaques was counted the next day to determine the titer.
8. Calculation of titer: Plates with a plaque number between 30-300 were selected. Titer was obtained by taking the average of two plates, multiplying the plaque number by the dilution fold and then multiplying by the elution volume.

Fc proteins were coated, and the phage display library was subjected to 6 rounds of depletion, and then incubated with streptavidin magnetic beads pre-bound with biotin-PDL1 to enrich PDL1-specific binding phages. Through four rounds of enrichment, the input and output of each round were counted, and the enrichment factor of each round was calculated.

As shown in Table 10, the enrichment factor (Input/output) indicates significant enrichment of the phage library, indicating the successful construction of the PDL1-specific binding phage library.

**Table 10**

| Round | Condition | Input (PFU) | Output (PFU) | Enrichment factor |
|---|---|---|---|---|
| First Round | Depletion antigen: 10 µg/mL human Fc | 9.95×10¹² | 1.96×10⁶ | 5.08×10⁶ |
| | Block: 20 µg/mL human Fc | | | |
| | Target antigen: 10 µg/mL biotin-BCMA + streptavidin magnetic beads | | | |
| | Washing conditions: 1000 µL PBS×10 | | | |
| | Elution conditions: 200 µL 1×TEA elution +100 µL 1M Tris-HCl neutralization | | | |
| Second Round | Depletion antigen: 10 µg/mL human Fc | 1.01×10¹³ | 1.88×10⁶ | 5.37×10⁶ |
| | Block: 20 µg/mL human Fc | | | |
| | Target antigen: 10 µg/mL biotin-BCMA + streptavidin magnetic beads | | | |
| | Washing conditions: 1000 µL PBS×10 | | | |
| | Elution conditions: 200 µL 1×TEA elution +100 µL 1M Tris-HCl neutralization | | | |
| Third Round | Depletion antigen: 10 µg/mL human Fc | 1.09×10¹³ | 6.21×10⁶ | 1.76×10⁶ |
| | Block: 20 µg/mL human Fc | | | |
| | Target antigen: 5 µg/mL biotin-BCMA + streptavidin magnetic beads | | | |
| | Washing conditions: 1000 µL PBS×10 | | | |
| | Elution conditions: 200 µL 1×TEA elution +100 µL 1M Tris-HCl neutralization | | | |
| Fourth Round | Depletion antigen: 10 µg/mL human Fc | 1.01×10¹³ | 1.92×10⁷ | 5.26×10⁵ |
| | Block: 20 µg/mL human Fc | | | |
| | Target antigen: 5 µg/mL biotin-BCMA + streptavidin magnetic beads | | | |
| | Washing conditions: 1000 µL PBS×10 | | | |
| | Elution conditions: 200 µL 1 TEA elution +100 µL 1M Tris-HCl neutralization | | | |

### Example 8. Cell-based panning of phage display library

The recombinant cell lines overexpressing the target protein were used. The phage library was sequentially incubated with empty cells and the cells overexpressing the target protein. After several washes to remove non-specifically binding phages, the recombinant phages bound to the cell surface were eluted by glycine or TEA and then amplified. After 3-4 rounds of panning, monoclonal colonies were selected for ELISA detection.
1. 1.5 mL EP tubes were blocked with 1% PBSA one day in advance, and placed at 4°C overnight.
2. 1×10⁷ of target cells and control cells were taken respectively, washed three times with PBS, resuspended in 10 mL of 1% PBSA, and placed on a decolorization shaker for low-speed blocking at room temperature for 1 hour, respectively.
3. 1×10¹¹ of phages were added to the control cells and incubated for 1 hour, while the target cells were continued to be blocked.
4. The incubated cells were centrifuged at 1000g for 5 minutes. The supernatant of the target cells (1% PBSA) was discarded, and the supernatant of the control cells was carefully aspirated and added to the target cell tube. After resuspension, the cells were placed on a shaker for low-speed incubation for 1 hour.
5. The target cells were centrifuged at 1000g for 5 minutes (while 1.5 mL EP tubes blocked one day in advance were washed three times with PBS). The supernatant of the target cells was discarded, 4 mL of PBS was added for resuspension, and the suspension was distributed into closed 1.5 mL EP tubes. The cells were centrifuged and washed five times with PBS, then transferred to four additional EP tubes for further 5 washes. Finally, the cells were transferred to the same EP tube.
6. The cells were resuspended in 200 µL of PBS, and then 200 µL of 2×TEA was added and pipetted quickly until the solution was no longer viscous. 200 µL of Tris-HCl was then added for neutralization, thereby obtaining the product.
7. The titer of the output library was determined, following the steps 7 and 8 in the solutionpanning scheme of Example 7.

The screening of the phage library can be performed using the methods described in Example 7 or Example 8 depending on the experimental conditions, and the present invention preferably uses the method in Example 7.

### Example 9. Phage ELISA

1. 2YT-Amp medium was dispensed into a 96-well deep-well plate with 500 µL per well. Mono clones from the output plate were picked into the wells and cultured at 37°C, 225 rpm until the OD600 reached 0.5. The last two wells, H11 and H12, were filled with only medium, serving as blank controls without clones.
2. Concurrently, the ELISA plate was coated with antigens using CBS at a concentration of 1 µg/mL, with 100 µL per well, and incubated at 37°C for 2 hours.
3. 2YT-A medium was dispensed into an additional 96-well deep-well plate with 500 µL per well. 10 µL of bacterial suspension with OD600 of 0.5 was transferred into the newly dispensed 96-well plate by using a multi-channel pipette, and cultured overnight at 37°C, 225 rpm. This bacterial suspension was the sample for sequencing.
4. M13KO7 was added to the bacterial suspension with OD600 of 0.5, mixed thoroughly, and allowed to stand at 37°C for 15 minutes.
5. Volume of M13KO7 = 10 × Volume × OD600 × 5 × 10⁸ / Titer of M13KO7.
6. The bacterial suspension after infection was placed on a shaker and cultured at 37°C, 225 rpm for 45 minutes.
7. The bacterial suspension was centrifuged at 4000 rpm for 10 minutes in a centrifuge. The supernatant was discarded, and the bacteria were resuspended in 800 µL per well of 2YT-AK medium. The plate was then placed back on the shaker and cultured overnight at 30°C, 210 rpm.
8. Concurrently, the antigens in the ELISA plate were removed, and the plate was washed three times with PBST wash buffer and blocked with 250 µL per well of 3% MPBS at 4°C overnight. An additional blank plate was also blocked as a BLANK control.
9. On the second day, the 96-well deep-well plate was centrifuged at 4000 rpm for 10 minutes in a centrifuge. The milk in the ELISA plate was discarded, and the plate was washed four times with 200 µL of PBST. 50 µL of PBST was added to each well, followed by the addition of 50 µL of centrifuged phage supernatant corresponding to each well. The plate was incubated at 4°C for 1 hour. The supernatant was discarded, and the plate was washed five times with PBST. HRP-Anti M13 secondary antibody was diluted with PBST and added to each well at 100 µL. After incubation at 4°C for 45 minutes, the secondary antibody was washed away. The plate was washed five times with PBST. TMB was added for color development at room temperature for 10 minutes, followed by termination with hydrochloric acid. The OD values of the clones in the plate were read, and clones with high S/N ratios were selected for sequencing using the preserved bacterial suspension.

The output products of the second and third rounds in Example 7 were selected for phage ELISA detection. Streptavidin, biotin-PDL1-Fc, and Fc proteins were coated separately, and the recombinant phage supernatant was used for ELISA detection, with directly blocked wells serving as the control group.

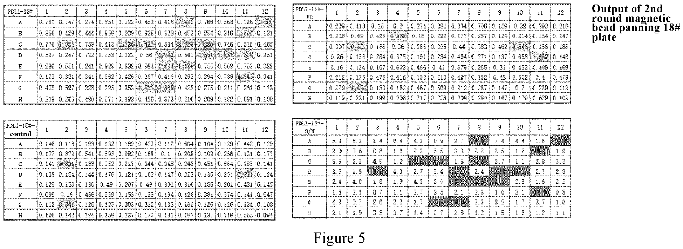

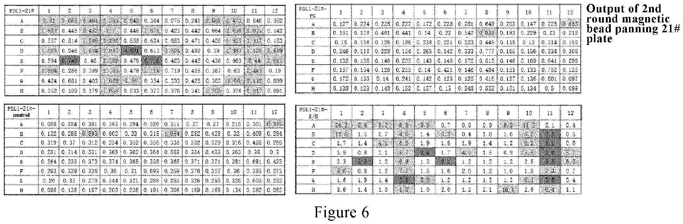

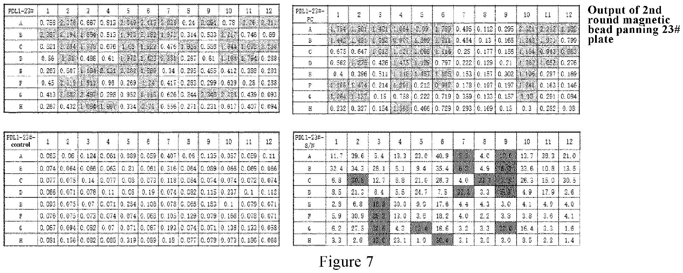

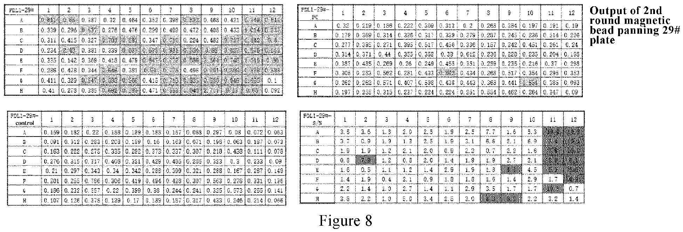

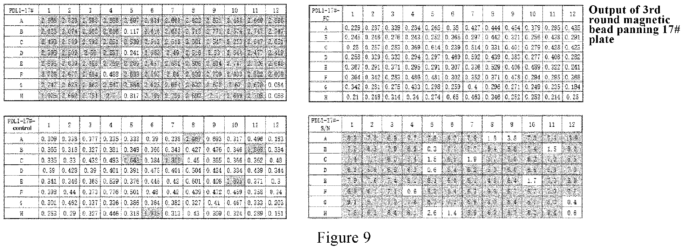

As shown in Figures 5-9, based on the ELISA detection results of the output products of the second and third rounds, clones that bind to both PDL1 antigen and Fc are marked in red; blue indicates clones that only bind to PDL1 target antigen and not to Fc; green indicates clones that only bind to PDL1 target antigen, while not to Fc, and show strong binding to PDL1 target antigen (i.e., clones sent for sequencing). Among them, clones with an S/N ratio above 4 and without binding to Fc were selected for sequencing and antibody sequence analysis.

After analysis, using streptavidin magnetic beads and biotinylated antigens for solution panning, a total of 6 different antibody sequences were obtained, namely 17-A06, 17-C12, 17-A01, 17-G06, 23-G3, and 29-H9 of the present invention.

Bacterial suspensions of the target clones were taken for PCR amplification, to add a signal peptide added to the N-terminus of VHH and IgG1-Fc to the C-terminus. The PCR products were transiently transfected into HEK293 cells, and supernatant containing the expressed antibody was taken for FACS detection.

### Example 10. Amplification, transient transfection, and detection of Overlap

### PCR products

1. First round PCR: amplification of CMV, VHH, and Fc
(1) The PCR reaction system [50 µL system/reaction] was prepared according to Table 11 (additional preparation can be made for CMV and Fc fragments) (Amplification primers for CMV fragments: CMV-F and PCom-R1; Amplification primers for VHH fragments: PCom-F1 and PCom-R2; Amplification primers for Fc fragments: PCom-F2 and PGK-R):

**Table 11**

| Component | Quantity |
|---|---|
| Upstream Primer (5 µM) | 2 µL |
| Downstream Primer (5 µM) | 2 µL |
| NuHi Power mix (2×) | 25 µL |
| Template (Plasmid or Bacterial suspension) | 1 µL |
| Sterile Water | Add to 50 µL |

Wherein the PCR reaction procedure was as follows:

| | | |
|---|---|---|
| 95°C | 10 min | |
| 95°C | 15 s | 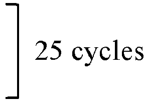 |
| 56°C | 30 s | |
| 68°C | 60 s | |
| 68°C | 10 min | |

(2) 50 µL of PCR product was taken, and 1/10 volume of 10 × loading buffer was added. Electrophoresis analysis was performed using 1% agarose. The band sizes of CMV and Fc were about 750 bp, while the band size of VHH was about 560 bp;
(3) The target bands were excised from the gel, and the PCR products were purified. The concentration was measured using NanoDrop (if the concentration is too high, it can be diluted for subsequent reactions).
2. Second round PCR: Overlap Extension PCR connecting CMV, VHH, and Fc
(1) The PCR reaction system was prepared according to Table 12:

**Table 12**

| Component | Quantity |
|---|---|
| CMV 1st product | 50 ng |
| VHH 1st product | 50 ng |
| Fc 1st product | 50 ng |
| NuHi Power mix (2×) | 25 µL |
| Sterile Water | Add to 46 µL |

Wherein the PCR reaction procedure was as follows:

| | | |
|---|---|---|
| 95°C | 10 min | |
| 95°C | 15 s | 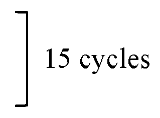 |
| 60°C | 30 s | |
| 68°C | 120 s | |

(2) After the PCR reaction procedure, 2 µL of each primer CMV-F and PGK-R were added, followed by the next PCR reaction procedure.
The next PCR reaction procedure was as follows:

| | | |
|---|---|---|
| 95°C | 15 s | 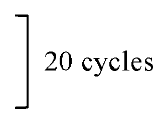 |
| 60°C | 30 s | |
| 68°C | 120 s | |
| 68°C | 10 min | |

(3) The overlap PCR product was purified using TakaRa's DNA Fragment Recovery Kit, and the concentration was measured using NanoDrop. At least 10 µg of PCR product was required for subsequent cell transfection verification;
(4) The transfection steps were the same as those for the transfection of eukaryotic expression vectors.

### Example 11. Transient transfection expression of single domain antibodies

1. LVTransm transfection reagent and antibody expression vector pcDNA3.4-hIgG I-Fc2 or the overlap PCR product were taken from the refrigerator, thawed at room temperature, and then thoroughly mixed with a pipette by pipetting up and down. PBS buffer was taken out and warmed to room temperature. 500 µL of PBS was taken into one well of a 24-well plate and added with 4 µg of pcDNA3.4-hIgG1-Fc2. After thorough mixing with a pipette by pipetting up and down, 12 µL of LVTransm was added, and immediately mixed again with a pipette by pipetting up and down. Then the plate was placed at room temperature for 10 minutes. The mixture here was called DNA/LVTransm complex.
2. The above 532 µL of DNA/LVTransm complex was added into 1.5 mL of 293F cells, gently shaken until well mixed. The cells were placed in a 37°C, 5% CO₂ incubator and cultured at 130 RPM for 6-8 hours. Then 1.5 mL of fresh 293 culture medium was added, and the cells were put back to the incubator for further cultivation.
3. After 3 days of continuous culture, the culture supernatant was collected by centrifugation and filtered with a 0.45 µm filter membrane, and the filtrate was transferred to a sterile centrifuge tube for following flow cytometry and ELISA detection.

### Example 12. Construction of VHH eukaryotic expression vectors

Based on the ELISA detection results of phage mono clones in Example 9, positive clones were selected for sequencing to obtain VHH antibody sequences. The obtained VHH antibody sequences were analyzed and synthesized respectively, and subcloned in tandem with human IgG1 Fc into the expression vector pcDNA3.4-hIgG1-Fc2. After the vectors were sequenced and confirmed to be correct, endotoxin-free plasmids were prepared by using Qiagen plasmid extraction kit for later use.

### Example 13. Flow cytometry detection of recombinant antibodies binding to target proteins

1. CHO-K1 cell strains and CHO-K1-PDL1 cell strains were thawed from liquid nitrogen and the cell state was adjusted to logarithmic growth phase.
2. Each of the two kinds of cells was divided into several sections with 5×10⁵ cells in each section.
3. The expressed antibodies were incubated with target cells CHO-K1 and CHO-K1-PDL1, respectively. After mixing well, the mixtures were incubated at room temperature for 1 hour.
4. The mixtures were centrifuged at 800×g at room temperature for 5 minutes, the supernatant containing antibodies was discarded, and the cells were washed with PBS for 3 times.
5. 1 µL of PE-labeled or Alexa Fluor 488-labeled Anti-human IgG was added. After mixing well, the mixtures were incubated at room temperature in the dark for 30 minutes.
6. The cells were centrifuged at 800×g at room temperature for 5 minutes, the supernatant containing the secondary antibodies was discarded, and the cells were washed with PBS for 3 times.
7. The cells were resuspended with 500 µL of PBS, and then subjected to flow cytometry.

CMV promoter, signal peptide, and human IgG1 Fc tag were added to the N-terminus and C-terminus of the candidate antibody sequences through Overlap PCR of Example 10, respectively. The purified PCR products were then transiently transfected into 293 cells as in Example 11, and the expressed antibodies were taken for flow cytometry detection.

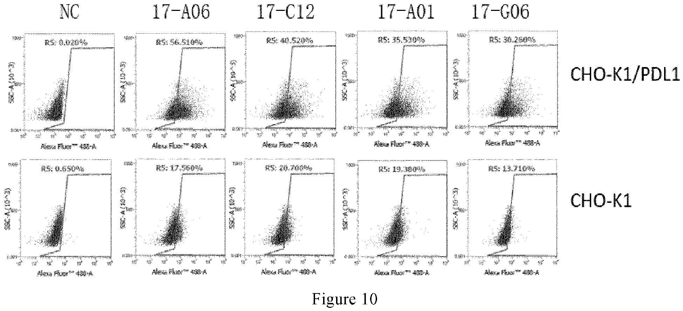

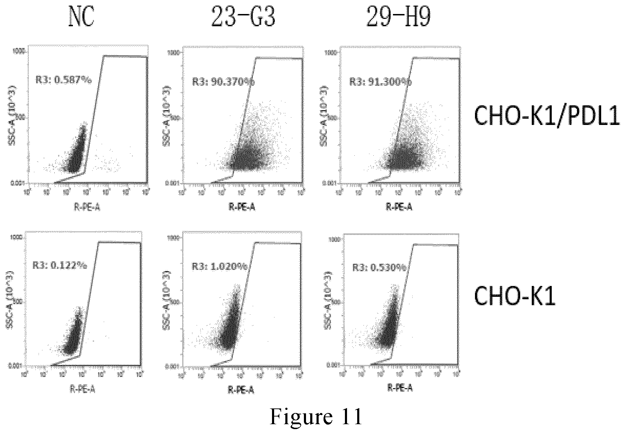

As shown in Figures 10 and 11, according to the FACS detection results, 17-A06, 17-C12, 17-A01, 17-G06, 23-G3, and 29-H9 specifically bind to the CHO-K1/PDL1 recombinant cell line, with little or no binding to CHO-K1.

These sequences were arranged for synthesis and were inserted into the expression vector pcDNA3.4-hIgG1-Fc2 for the expression and purification of the candidate antibodies.

### Example 14. Expression and purification of antibodies

1. LVTransm transfection reagent and single chain antibody expression vectors were taken from the refrigerator, thawed at room temperature, and then thoroughly mixed with a pipette by pipetting up and down. PBS or HBSS buffer was taken out and warmed to room temperature. 2 mL of PBS was taken into one well of a 6-well plate and added with 130 µg of pcDNA3.4-hIgG1-Fc2. After thorough mixing with a pipette by pipetting up and down, 400 µL of LVTransm was added, and immediately mixed again with a pipette by pipetting up and down. Then the plate was placed at room temperature for 10 minutes.
2. The above DNA/LVTransm complex was added into 50 mL of 293F cells, gently shaken until well mixed. The cells were placed in a 37°C, 5% CO₂ incubator and cultured at 130 RPM for 6-8 hours. Then 50 mL of fresh 293 culture medium was added, and the cells were put back to the incubator for further cultivation.
3. After 7 days of continuous culture, the culture supernatant was collected by centrifugation and filtered with a 0.45 µm filter membrane, and the filtrate was transferred to a sterile centrifuge tube. The antibodies were purified using a Protein A column.

The expression vectors for candidate PDL1 antibodies (i.e., 17-A06, 17-C12, 17-A01, 17-G06, 23-G3, and 29-H9) were transiently transfected into 293F cells, and the transfected supernatants were collected. The candidate antibodies were then purified using protein A.

The candidate antibodies were diluted in four gradients, starting from 10 µg/mL and with 5-fold dilutions. Flow cytometry was used to detect the binding of the purified antibodies to CHO-K1/PDL1, a recombinant cell line overexpressing PDL1.

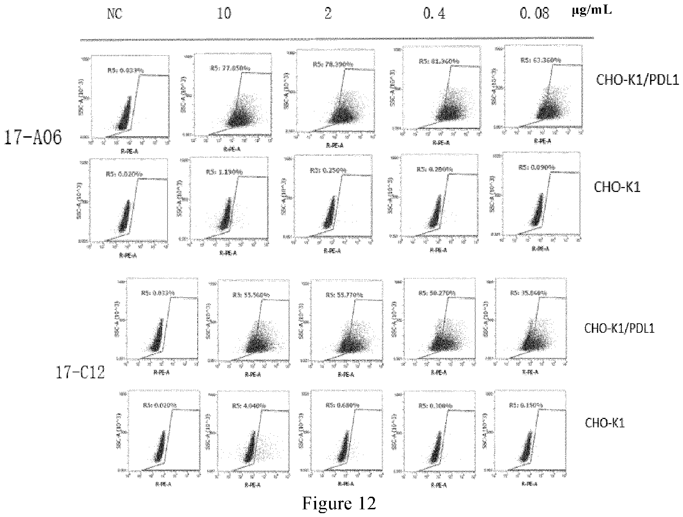

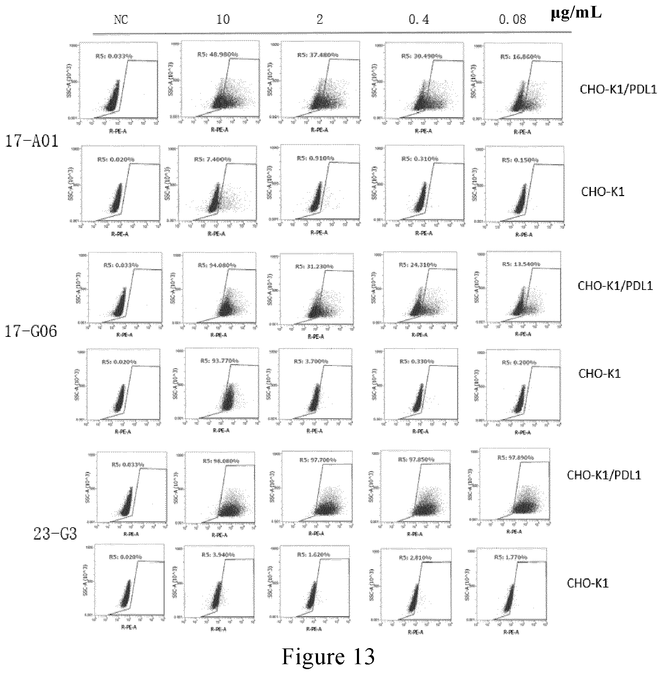

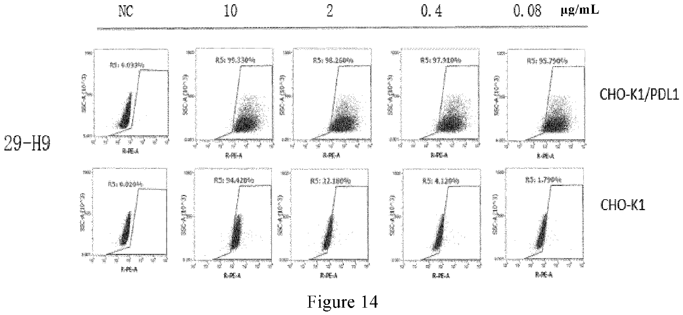

The flow cytometry results shown in Figures 12-14 indicate that as the concentration of the candidate antibodies gradually decreases, the binding of the candidate antibodies to the PDL1-overexpressing cell line also changes gradually, suggesting that the purified 17-A06, 17-C12, 17-A01, 17-G06, 23-G3, and 29-H9 antibodies can all specifically bind to the PDL1-overexpressing cell line.

### Example 15. Detection of PD1-PDL1 blocking activity

The Jurkat-PD1-NFAT-Luc reporter gene cell line and ^{aAPC}CHO-PDL1 cells were thawed and continuously passaged until logarithmic growth phase. In a 96-well plate, effector cells Jurkat-PD1-NFAT-Luc were inoculated at 2×10⁴ cells per well, and target cells ^{aAPC}CHO-PDL1 were added at a ratio of 1:1. Gradient-diluted detection antibodies (positive antibody: Nivolumab; antibodies to be tested) were added to corresponding wells, with a 3-fold gradient dilution applied for a total of 9 dilutions which resulted in final concentrations of 30 µg/mL, 10 µg/mL, 3.333 µg/mL, 1.111 µg/mL, 0.3704 µg/mL, 0.1235 µg/mL, 0.04115 µg/mL, 0.01372 µg/mL, and 0.004572 µg/mL. After co-culturing for 18 hours, 25 µL of One-Glo reagent was added to each well, and the luciferase activity values in the wells were measured using a Tecan M1000pro microplate reader.

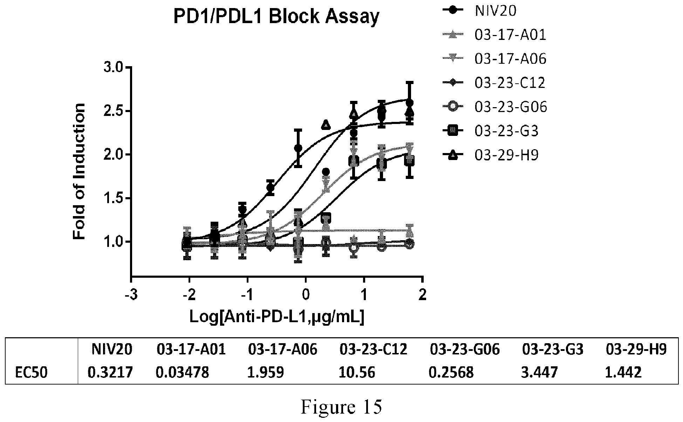

As shown in Figure 15, the Nivolumab positive control can block the PD-1/PDL1 interaction with an EC50 of 0.3217 µg/mL. Among the antibodies to be tested, the PDL1 single domain antibodies 17-A06, 23-G3, and 29-H9 can all block the PD-1/PDL1 interaction, while the other three candidate antibodies do not possess blocking capabilities. However, the EC50 values of the PDL1 candidate antibodies 17-A06, 23-G3, and 29-H9 were higher than that of the positive control antibody Nivolumab, presumably due to the smaller size of the single domain antibodies and their limited epitope occupancy. The three antibodies with blocking function were arranged to randomly combine with each other to construct bivalent antibodies, and then the bivalent antibodies were arranged for detection of blocking activity.

The three candidate antibodies were randomly combined to construct bivalent antibodies in the forms of 17-A06-G4S-23-G3, 17-A06-G4S-29-H9, and 23-G3-G4S-29-H9. After purifying the antibodies, the PD-1-PDL1 blocking activity of the bivalent antibodies was tested using Jurkat-PD-1-NFAT-Luc reporter gene cell lines and ^{aAPC}CHO-PDL1 cells.

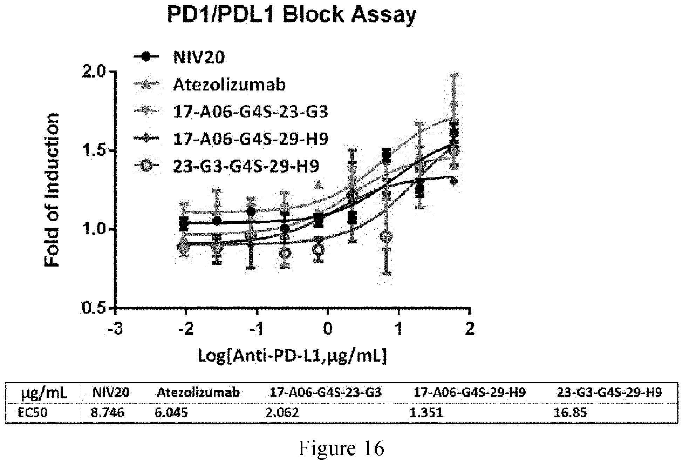

As shown in Figure 16, the Nivolumab (PD-1 antibody, NIV20) positive control and Atezolizumab (PDL1 antibody) can both block the PD-1/PDL1 interaction, with comparable EC50 values. The tested bivalent PDL1 antibodies were all able to block the PD-1/PDL1 interaction, and their blocking capabilities were consistent with the positive control.

### Example 16. Affinity detection of recombinant antibodies

The PDL1 candidate antibodies (Fc-tagged) were immobilized on protein A probes at a concentration of 5 µg/mL, and affinity detection was performed using the OCTET R2 with gradient dilutions of PDL1-His as the mobile phase, with final concentrations of 2.6 µg/mL, 1.3 µg/mL, 0.65 µg/mL, 0.325 µg/mL, and 0.1625 µg/mL.

The affinity detection was conducted on the three bivalent PDL1 antibodies using the OCTET R2, and the results are presented in Table 13.

**Table 13**

| Bivalent antibody | kon | koff | KD |
|---|---|---|---|
| 17-A06-G4S-23-G3 | 1.547×10⁵ M⁻¹S⁻¹ | 6.493×10⁻⁴ S⁻¹ | 4.198×10⁻⁹ M |
| 17-A06-G4S-29-H9 | 1.760×10⁵ M⁻¹S⁻¹ | 4.804×10⁻⁴ S⁻¹ | 2.729×10⁻⁹ M |
| 23-G3-G4S-29-H9 | 2.378×10⁵ M⁻¹S⁻¹ | 4.554×10⁻⁴ S⁻¹ | 1.915×10⁻⁹ M |

The detection results show that the affinity of the three bivalent antibodies was very high, ranging from high to low at 4.198×10⁻⁹M, 2.729×10⁻⁹M, and 1.915×10⁻⁹M, respectively.

All documents mentioned in the present invention are incorporated by reference herein as each document is incorporated separately by reference. Furthermore, it should be understood that after reading the foregoing teachings of the present invention, various changes or modifications may be made to the present invention by those skilled in the art and that these equivalents are equally within the scope of the claims appended to this application.

## Claims

1. A PDL1-targeting nanobody, wherein the complementarity determining regions (CDRs) of the VHH chain of the PDL1-targeting nanobody is one or more selected from the group consisting of:
(1) CDR1 as shown in SEQ ID NO: 7, CDR2 as shown in SEQ ID NO: 8, and CDR3 as shown in SEQ ID NO: 9;
(2) CDR1 as shown in SEQ ID NO: 10, CDR2 as shown in SEQ ID NO: 11, and CDR3 as shown in SEQ ID NO: 12;
(3) CDR1 as shown in SEQ ID NO: 10, CDR2 as shown in SEQ ID NO: 13, and CDR3 as shown in SEQ ID NO: 14;
(4) CDR1 as shown in SEQ ID NO: 10, CDR2 as shown in SEQ ID NO: 13, and CDR3 as shown in SEQ ID NO: 15;
(5) CDR1 as shown in SEQ ID NO: 7, CDR2 as shown in SEQ ID NO: 16, and CDR3 as shown in SEQ ID NO: 17;
(6) CDR1 as shown in SEQ ID NO: 18, CDR2 as shown in SEQ ID NO: 19, and CDR3 as shown in SEQ ID NO: 20.

2. The PDL1-targeting nanobody of claim 1, wherein the VHH chain of the PDL1-targeting nanobody further comprises framework regions (FRs), wherein the framework regions (FRs) is one or more selected from the group consisting of:
(1) FR1 as shown in SEQ ID NO: 21, FR2 as shown in SEQ ID NO: 22, FR3 as shown in SEQ ID NO: 23, and FR4 as shown in SEQ ID NO: 24;
(2) FR1 as shown in SEQ ID NO: 25, FR2 as shown in SEQ ID NO: 26, FR3 as shown in SEQ ID NO: 27, and FR4 as shown in SEQ ID NO: 24;
(3) FR1 as shown in SEQ ID NO: 28, FR2 as shown in SEQ ID NO: 29, FR3 as shown in SEQ ID NO: 30, and FR4 as shown in SEQ ID NO: 24;
(4) FR1 as shown in SEQ ID NO: 31, FR2 as shown in SEQ ID NO: 29, FR3 as shown in SEQ ID NO: 32, and FR4 as shown in SEQ ID NO: 24;
(5) FR1 as shown in SEQ ID NO: 33, FR2 as shown in SEQ ID NO: 22, FR3 as shown in SEQ ID NO: 34, and FR4 as shown in SEQ ID NO: 24;
(6) FR1 as shown in SEQ ID NO: 35, FR2 as shown in SEQ ID NO: 36, FR3 as shown in SEQ ID NO: 37, and FR4 as shown in SEQ ID NO: 24.

3. The PDL1-targeting nanobody of claim 1, wherein the amino acid sequence of the VHH chain of the PDL1-targeting nanobody is selected from the group consisting of: SEQ ID NO: 1, SEQ ID NO: 2, SEQ ID NO: 3, SEQ ID NO: 4, SEQ ID NO: 5, SEQ ID NO: 6, and a combination thereof.

4. A PDL1-targeting antibody, which comprises one or more of the PDL1-targeting nanobody of claim 3.

5. The PDL1-targeting antibody of claim 4, wherein the antibody comprises a monomer, a bivalent antibody, and/or a multivalent antibody

6. A polynucleotide encoding a protein selected from the group consisting of: the PDL1-targeting nanobody of claim 1, and the PDL1-targeting antibody of claim 4.

7. An expression vector comprising the polynucleotide of claim 6.

8. A host cell comprising the expression vector of claim 7, or having the polynucleotide of claim 6 integrated into its genome.

9. A method for producing a PDL1-targeting nanobody, which comprises the steps of:
(a) culturing the host cell of claim 8 under conditions suitable for producing a nanobody, thereby obtaining a culture containing the PDL1-targeting nanobody;
(b) isolating and/or recovering the PDL1-targeting nanobody from the culture; and
(c) optionally, purifying and/or modifying the PDL1-targeting nanobody obtained in step (b).

10. An immunoconjugate, which comprises:
(a) the PDL1-targeting nanobody of claim 1, or the PDL1-targeting antibody of claim 4; and
(b) a coupling moiety selected from the group consisting of: a detectable label, a drug, a cytokine, a radionuclide, an enzyme, a gold nanoparticle/nanorod, a nanomagnetic particle, a viral coat protein or VLP, and a combination thereof.
